(19) Europäisches Patentamt
European Patent Office
Office européen des brevets

(11) **EP 4 296 350 A1**

(12) **EUROPEAN PATENT APPLICATION**

(43) Date of publication:
**27.12.2023 Bulletin 2023/52**

(21) Application number: **22180917.1**

(22) Date of filing: **24.06.2022**

(51) International Patent Classification (IPC):
*C12M 1/36* (2006.01)          *G06N 3/04* (2023.01)
*G06N 3/08* (2023.01)          *G05B 13/02* (2006.01)
*G16B 5/00* (2019.01)          *G16B 40/00* (2019.01)

(52) Cooperative Patent Classification (CPC):
**G16B 5/30; G05B 17/02; G06N 3/08; C12M 41/48**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Yokogawa Insilico Biotechnology GmbH**
**70563 Stuttgart (DE)**

(72) Inventors:
• **KIRCH, Jakob Simon**
  **70563 Stuttgart (DE)**

• **EßWEIN, Sabrina Karin**
  **70563 Stuttgart (DE)**
• **SCHINDLER, Kevin Michael**
  **70563 Stuttgart (DE)**
• **CHHABRA, Pulkit**
  **139950 Singapore (SG)**
• **SCHMID, Joachim Walter**
  **70563 Stuttgart (DE)**
• **NABER, Maike**
  **70563 Stuttgart (DE)**
• **MAUCH, Klaus Jürgen**
  **70563 Stuttgart (DE)**

(74) Representative: **2SPL Patentanwälte PartG mbB**
**Landaubogen 3**
**81373 München (DE)**

(54) **A CONCEPT FOR TRAINING AND USING AT LEAST ONE MACHINE-LEARNING MODEL FOR MODELLING KINETIC ASPECTS OF A BIOLOGICAL ORGANISM**

(57)     Examples relate to a concept for training and using at least one machine-learning model for modelling kinetic aspects of a biological organism, and in particular to a method, apparatus, and computer program for training the at least one machine-learning model for modelling the kinetic aspects of the biological organism, and various methods using such a trained at least one machine-learning model. The method for training the at least one machine-learning model for modelling the kinetic aspects of the biological organism comprises training the machine-learning model based on training data. The training data is based on experimental data of a plurality of clones of the biological organism. The training data comprises a subset of training data that is based on experimental data of a single clone. A first component of the at least one machine-learning model is trained using the training data, with the first component representing a generic kinetic behavior of the biological organism. A second component of the at least one machine-learning model is trained using the subset of the training data, the second component representing a clone-specific kinetic behavior of the biological organism.

Fig. 1

**Description**

**Field**

Examples relate to a concept for training and using at least one machine-learning model for modelling kinetic aspects of a biological organism, and in particular to a method, apparatus, and computer program for training the at least one machine-learning model for modelling the kinetic aspects of the biological organism, and various methods using such a trained at least one machine-learning model.

**Background**

Today, Digital Twins are used in various industrial fields, for example in the automotive industry, since Digital Twins significantly improve and speed-up design, optimization and control of machines, industrial products, and supply chains. Through their predictive qualities, Digital Twins can be used to intervene directly in production or to predict and improve the overall behavior of assets and the supply chain. This facilitates the monitoring and optimization of processes as well as the control to increase process robustness, product yield and quality.

Despite such advantages, Digital Twins are rarely applied in biotechnological production processes. The main reasons lie in the limited applicability of Digital Twins for different organisms and cell lines, the process setup and scale as well as the high requirements regarding measurement data. For instance, during manufacturing, only a limited amount of data can be obtained. Often not all required measurement entities are available that were previously used for the generation of a Digital Twin with high predictive quality. Measurement noise may further limit the predictive quality of the models, which is desired for robust process control.

Although mathematical models of biotechnological processes have been developed in the past, most of these models cannot tackle different application cases, e.g., in terms of clones, products and process formats. For each new application case, new data and models need to be generated. This leads to significant costs in resources and time. On the other hand, process optimization is primarily done via simple design of experiments and control via the glucose profile as well as other quantities such as pH and temperature due to the lack of suitable models or software platforms to enable the automated integration of data as well as generation and application of high-quality models. The applicability of such models is limited because the predictive quality might not be considered sufficient with the reduced data and quality that is observed for instance during manufacturing. Thus, they often cannot be used for process monitoring and robust control.

There may be a desire for an improved concept for a Digital Twin for use in biotechnological production processes.

**Summary**

This desire is addressed by the subject-matter of the independent claims.

Various examples of the present disclosure are based on the finding, that the lack of experimental data necessary for the generation of a Digital Twin can be overcome by using experimental data across clones, cell lines and/or process formats to generate a generalized Digital Twin, which can then additionally be adjusted to a specific clone of a cell line. Due to the additional experimental data available, such a Digital Twin may yield a higher predictive quality than smaller, cell-line-, clones- and process-format-specific Digital Twins. In the proposed concept, the generation of such a Digital Twin includes the training of at least one machine-learning model, which comprises a first component (e.g., a first machine-learning model or a first plurality of layers of a neural network) that is trained to model generic (i.e., not clone-specific) kinetic aspects of the biological organism being modeled, and a second component (e.g., a second machine-learning model or a second plurality of layers of a neural network) that is trained to model clone-specific aspects of the biological organism. For this training, training data is used, with only a clone-specific subset being used to train the second, clone-specific component. Once trained, the trained parameters may be re-used for the integration of new data sets, such that the Digital Twin can be extended and tuned to specific clones with reduced computational costs and reduced data requirements. These Digital Twins can be used offline for process improvement or optimization (including clone selection, platform media design) as well as online for monitoring and control of biotechnological processes to improve process robustness, performance, and product quality. Thus, the presented Digital Twin may for instance be used during manufacturing for the improvement of product quality or for process monitoring as well as control since its high predictive quality may improve the estimation of relevant system states despite measurement uncertainties.

The proposed concept may thus address one or more of the above-mentioned limitations by reusing data and model parameters from previous cultivation runs in order to reduce data requirements for the generation of Digital Twins for new application cases, including different clones or process scales while maintaining high predictive quality. The Digital Twin may be created via combining generic and clone-specific machine-learning models (i.e., the aforementioned first and second component), e.g., neural network(s), with metabolic functionalities as well as a reactor model that can be adapted for different process setups and scales via a hybrid approach (e.g., as shown in WO 2020/224779 A1). The machine-learning model(s) contain generic as well as clone-specific parameters (i.e., the aforementioned first and second component) that can be trained together or separately depending on the application specification. This leads to a broad applicability of the approach for different cell lines, clones, and scales. The generic parameters can be trained on all of the datasets and conserve generic metabolic behavior. On the other hand, the clone-specific parameters are only trained on the dataset for a certain clone (clone-specific) and can thus learn the clone-specific behavior beyond the generic behavior. The Digital Twin that is based on these machine-learning model(s) may learn generic and clone-specific behavior and can be embedded in a control strategy, including state estimation and model predictive control. The presented Digital Twin can, for instance, be used during manufacturing for the improvement of product quality or for process monitoring as well as control since its high predictive quality allows the estimation of relevant system states despite measurement uncertainties.

Various aspects of the present disclosure relate to a method for training at least one machine-learning model for modelling kinetic aspects of a biological organism. For example, the at least one machine-learning model may be suitable for a Digital Twin of the biological organism or of a bioreactor comprising the biological organism. The method comprises training the machine-learning model based on training data. The training data is based on experimental data of a plurality of clones of the biological organism. The training data comprises a subset of training data that is based on experimental data of a single clone. A first component of the at least one machine-learning model is trained using the (entire) training data. The first component represents a generic kinetic behavior of the biological organism. A second component of the at least one machine-learning model is trained using the subset of the training data. The second component represents a clone-specific kinetic behavior of the biological organism. By training both a first component representing generic kinetic behavior (that is common to different clones, process scales and/or cell-lines) and a second component representing clone-specific behavior, additional experimental data can be used to train the first component with an increased predictive quality, which is fine-tuned to the specific clone of interest through the use of the second component. This may result in the machine-learning model(s) having an overall increased predictive quality, and thus an increased predictive quality of a Digital Twin employing such machine-learning model(s).

In general, e.g., in supervised learning-based training approaches, the training data comprises training input data and training output data. For example, the training input data may comprise a representation of an experimental environment of the organism. The training output data may represent kinetic properties (e.g., concentrations, changes in concentrations or (flow) rates) observed in response to the respective experimental environment. Thus, the data that is commonly sampled during experiments involving biological organisms in a bioreactor may be used for the training of the at least one machine-learning model, e.g., with some pre-processing to generate the inputs (and outputs) expected by the at least one machine-learning model.

Training a machine-learning model often involves changing the machine-learning model such, that a difference between observed output of the machine-learning model and an expected output of the machine-learning model (e.g., as defined by the training output data) decreases over time during the training. Accordingly, training the at least one machine-learning model may comprise determining a deviation between an output of a function and the training output data, with the function being based on the at least one machine-learning model, a first set of flux modes representing generic functionality of the plurality of clones of the biological organism and a second set of flux modes specific to the single clone of the biological organism. For example, the output(s) of the at least one machine-learning model may be used as parameters of the function, along with the first and second set of flux modes, and then compared with the training output data, or the training output data may be pre-processed using the first and second set of flux modes and then compared with the output(s) of the at least one machine-learning model. In a specific example, the training output data may be compared with a hybrid model (e.g., as discussed in WO 2020/224779 A1), which may be based on the at least one machine-learning model, the first and second set of flux modes, and optionally based on one or more other models, such as a reactor model. For example, the flux mode may be both elementary flux modes (EFMs) and metabolic base functionalities (also denoted base modes). For example, for the generation of the metabolic base functionalities (such as biomass production, product formation or production of non-essential amino acids) flux balance analysis may be applied. Further functionalities may be complemented by elementary flux mode analysis.

In some examples, the representation of the experimental environment (contained in the training input data) may comprise more information than necessary for training purposes. Therefore, dimensionality reduction may be applied to the representation of the experimental environment. For example, the representation of the experimental environment may correspond to a compressed representation of the experimental environment having a reduced dimensionality compared to an uncompressed representation of the experimental environment. This may harmonize the input vector across different sets of data representing the experimental environment and also reduce the complexity, and thus computing power required, of the training.

In many cases, the envisioned Digital Twins are used to improve a production process of a biological component of a pharmaceutical product. Therefore, the quality of the biological component may be another factor that the machine-learning model(s) may be trained on. For example, the training output data may further represent one or more bio-pharmaceutical product quality properties of the biological organism in an experimental environment. In effect, the at least one machine-learning model, and the Digital Twin that is based on the at least one machine-learning model, may also be used to make predictions on the quality of the biological component being produced, and may thus be used to select the environment of the biological organism such, that the quality of the bio-pharmaceutical product is improved.

As outlined above, the training data is based on experimental data from multiple clones. In some examples, the multiple clones may be clones of the same cell-line. In other words, the training data may be based on experimental data of a plurality of clones of the same cell-line of the biological organism. Using multiple clones of the same cell-line may improve the homogeneity of the experimental results, and thus the predictive quality, albeit based on a more limited pool of training data. Alternatively, the training data may be based on experimental data of a plurality of clones of a plurality of different cell-lines of the biological organism.

This may increase the pool of training data, which may increase the predictive quality, but may lead to problems (e.g., with the at least one machine-learning model converging) if the cell-lines do not behave similar enough.

Moreover, the training data may be based on experimental data from a plurality of different process scales (e.g., from small experimental setups to full scale production). This may improve the predictive qualities of the at least one machine-learning model when applied to predict the kinetic behavior of the biological organism at different process scales.

For example, the at least one machine-learning model may comprise at least one deep neural network, with the first component comprising a first plurality of layers of the at least one deep neural network and the second component comprising a second plurality of layers of the at least one deep neural network. For example, the first and second component may be implemented as different layers of the same neural network, or as two separate neural networks.

According to an example, the first component and the second component may be trained in a first phase of the training and the second component may be trained in a second phase of the training following the first phase of the training, with the first component being frozen during the second phase of the training. In general, additional, clone-specific training might only provide limited benefits to the first component, so the first component may be frozen to decrease the training complexity.

In various examples, the first and second component are trained based on an output of the respective other component. For example, the training output data may be compared with a combination of the respective outputs of the first and second component, such that the output of both components have an influence on the training of the respective other component.

In some examples, the at least one machine-learning model further may comprise a third component taking an output of the first and second component at its inputs. The method may comprise training the third component of the at least one machine-learning model using the training data. This third component may be used to combine the outputs of the first and second component, for example. Alternatively, the outputs may be combined (e.g., multiplied) as part of a (deterministic) function.

In various examples, the at least one machine-learning model further may comprise a fourth component representing one or more flux modes not represented by the first and/or second component. The method may comprise training the fourth machine-learning model using the training data. The fourth machine-learning model may thus add support for clone-specific flux modes not represented by the first and second machine-learning model.

In various examples, the at least one machine-learning model may be trained using a stochastic algorithm. For example, the so-called ADAM algorithm, which may be considered to be a stochastic gradient descent-type algorithm, may be used.

According to an example, the at least one machine-learning model may form a set of machine-learning models. For example, the method may comprise training a plurality of sets of machine-learning models. The plurality of sets of machine-learning models may be trained with different seed values, with the different seed values affecting at least one of a random initialization of parameters and a dropout of the respective machine-learning models. This approach is denoted the "ensemble method" and can be used to assess the uncertainty in predictive models, by comparing and/or combining the results generated by multiple different machine-learning models having received the same training (albeit with random differences in starting parameters and/or dropout).

In some examples, the method further comprising adapting, using transfer learning, at least the clone-specific second component of the least one machine-learning model based on training data that is based on experimental data of a further single clone. This way, the computational effort already invested in generating a machine-learning model(s) for modeling a first clone may be re-used to generate a further machine-learning model(s) for modeling a second clone.

According to an example, the first and second component of the at least one machine-learning model are separate machine-learning models. Alternatively, the first and second component of the at least one machine-learning model may be a first and a second plurality of layers of the same deep neural network. Both approaches are suitable in the present context.

As outlined above, the training of the at least one machine-learning model may be part of a process of generating a Digital Twin that can be used to model the behavior of the biological organism (e.g., a bioreactor comprising the biological organism). Accordingly, the method may further comprise generating a Digital Twin of the biological organism using the trained at least one machine-learning model. This Digital Twin may be used for a number of different purposes, as will become evident in the following.

For example, the Digital Twin may be used for the purpose of experiment design. For example, the method may comprise determining a plurality of experiments to be performed using the biological organism. For example, the experiments may be determined such, that environmental conditions previously not studied are covered, or such that environmental conditions which lead to less accurate predictions are studied in greater detail. Once the experiments have been conducted, the resulting experimental data may be used to continue the training, and thus improve the predictive quality of the at least one machine-learning model, and thus of the Digital Twin. In other words, the method may comprise continuing training of the at least one machine-learning model based on further training data that is based on the plurality of experiments.

Another application of the generated Digital Twin is the determination of target parameters, such as media composition, feeding strategy, etc., for a bioreactor comprising the biological organism. Some aspects of the present disclosure thus relate to a method for determining at least one target parameter of at least one bioreactor comprising at least one biological organism (i.e., each bioreactor comprising a biological organism) . The method comprises determining the at least one target parameter using at least one Digital Twin of the at least one biological organism that is generated according to the above method and at least one corresponding cost function. For example, the at least one target parameter may comprise at least one of a target media composition of a feed medium for the at least one biological organism, a target feeding strategy for the at least one biological organism, a target outflux strategy for the at least one biological organism, and target initial conditions for the at least one biological organism. Due to the improved predictive quality of the Digital Twin, the quality of the determined target parameters may also be improved. For example, the at least one target parameter may be commonly determined for at least two biological organisms using at least two Digital Twins of the at least two biological organisms, e.g., for a platform media commonly used for multiple different biological organisms.

Moreover, such Digital Twins may be used to compare properties of different clones, e.g., for the purpose of clone selection. Some aspects of the present disclosure thus relate to a method for selecting a clone of a biological organism. The method comprises generating a plurality of Digital Twins of a plurality of clones of the biological organism using the above method. The method comprises selecting the clone by comparing one or more properties of the plurality of Digital Twins. Again, the improved predictive quality of the Digital Twin may also improve the clone selection process.

Two further applications of such Digital Twins relate to monitoring and controlling a biological manufacturing process. For example, some aspects of the present disclosure relate to a method for monitoring a biological manufacturing process involving a biological organism. The method comprises determining an estimated state of the biological manufacturing process using a Digital Twin of the biological organism that is generated according to the above method. For example, the Digital Twin may be supplied with information on the environment of the biological organism using a moving horizon-approach. The method comprises comparing the estimated state of the biological manufacturing process with an observed state of the biological manufacturing process. This way, unexpected occurrences may be detected during the manufacturing process.

For example, a moving-horizon estimation algorithm may be used in the comparison of the estimated state of the biological manufacturing process with the observed state of the biological manufacturing process. This may enable the estimation of unknown parameters, such as unknown metabolite concentrations, over time.

As outlined above, some aspects of the present disclosure relate to a method for controlling a biological manufacturing process involving a biological organism. The method comprises continuously adapting an environment of the biological manufacturing process using a Digital Twin of the biological organism that is generated according to the above method. The Digital Twin is supplied with information on the environment of the biological organism using a receding horizon approach. The method comprises comparing an estimated state of the biological manufacturing process with a defined reference state trajectory of the biological manufacturing process. For example, the difference between the estimated state and the defined reference state trajectory may be used to perform the continuous adjustment, e.g., to change the state according to the state trajectory.

Various aspects of the present disclosure relate to one or more computer systems comprising processing circuitry and storage circuitry, with the computer system being configured to perform at least one of the above methods.

Similarly, various aspects of the present disclosure relates to computer programs having a program code for performing at least one of the above methods, when the computer program is executed on a computer, a processor, or a programmable hardware component.

**Brief description of the Figures**

Some examples of apparatuses and/or methods will be described in the following by way of example only, and with reference to the accompanying figures, in which

Fig. 1              shows a schematic representation of a flux decomposition algorithm according to an example;

Figs. 2a and 2b     show flow charts of examples of a method for training at least one machine-learning model for modelling kinetic aspects of a biological organism;

Fig. 2c             shows a schematic diagram of an example of a computer system;

Fig. 3              shows a flow chart of an example of a method for determining at least one target parameter;

Fig. 4              shows a flow chart of an example of a method for monitoring a biological manufacturing process involving a biological organism;

Figs. 5a and 5b     show schematic diagrams of a moving horizon estimation algorithm according to an example;

Fig. 6              shows a flow chart of an example of a method for controlling a biological manufacturing process involving a biological organism;

Figs. 7a and 7b     show schematic diagrams of a model predictive control algorithm according to an example;

Figs. 8a and 8b     show schematic diagrams of a model predictive monitoring and control algorithm;

Fig. 9              shows a flow chart of an example of a method for selecting a clone of a biological organism;

Fig. 10             shows regression plots showcasing a predictive quality of an example;

Fig. 11             shows a schematic comparison of predictions with and without state estimation as well as corresponding measurement results; and

Fig. 12             shows a schematic diagram showcasing a process which deviated from a golden batch.

## Detailed Description

Some examples are now described in more detail with reference to the enclosed figures. However, other possible examples are not limited to the features of these embodiments described in detail. Other examples may include modifications of the features as well as equivalents and alternatives to the features. Furthermore, the terminology used herein to describe certain examples should not be restrictive of further possible examples.

Throughout the description of the figures same or similar reference numerals refer to same or similar elements and/or features, which may be identical or implemented in a modified form while providing the same or a similar function. The thickness of lines, layers and/or areas in the figures may also be exaggerated for clarification.

When two elements A and B are combined using an "or", this is to be understood as disclosing all possible combinations, i.e., only A, only B as well as A and B, unless expressly defined otherwise in the individual case. As an alternative wording for the same combinations, "at least one of A and B" or "A and/or B" may be used. This applies equivalently to combinations of more than two elements.

If a singular form, such as "a", "an" and "the" is used and the use of only a single element is not defined as mandatory either explicitly or implicitly, further examples may also use several elements to implement the same function. If a function is described below as implemented using multiple elements, further examples may implement the same function using a single element or a single processing entity. It is further understood that the terms "include", "including", "comprise" and/or "comprising", when used, describe the presence of the specified features, integers, steps, operations, processes, elements, components and/or a group thereof, but do not exclude the presence or addition of one or more other features, integers, steps, operations, processes, elements, components and/or a group thereof.

In the present disclosure, the term "optimization", "optimize", "optimal", "maximum", "minimum" etc. are used in a non-absolutist manner. If something is optimized, the result is not necessarily the optimal result, but the best or one of the best results given constraints such as limited runtime etc. Therefore, even the "optimal" result may not necessarily represent the absolute optimum, but the best or one of the best results given the above-referenced constraints. In the present disclosure, an optimization refers to the process of finding ever better results, not to the determination of a single best result.

Various examples of the present disclosure relate to a method for reusing data across cell lines, clones and projects for biotechnological process optimization and control. It provides a new method for the automatic generation, validation, and simulation of Digital Twins, e.g., across cell lines, process formats (i.e., fed-batch and perfusion), scales and products and its application for the purpose of optimization, scale-up, monitoring and control of biotechnological production processes. Some examples of the present proposed concept relate to cell culture methods and compositions thereof that in one aspect improve the yield and quality of a biologic therapeutic protein.

In the following, a short introduction is given to Digital Twins and metabolic network models (of biological organisms). Digital Twins, such as the Insilico Digital Twin, often use a metabolic network model which was constructed based on biological knowledge. This may ensure that (all) intracellular and extracellular fluxes are consistent with the available pathways and transformation steps of the given organism at any time point of a simulation. This may include that the directionality of irreversible reaction steps are considered. Mass balances (i.e., elemental balances and charge balances) may be closing for (all of) the time points during a simulation with the Digital Twin. In the following, a mathematical representation of a metabolic network model is explained in detail.

A metabolic network model is a mathematical representation of biochemical pathways in an organism. A pathway is a sequence of biochemical reactions in which a set of substrate metabolites is transformed into a set of products. Typical pathways of a metabolic network may include one or more of glycolysis, pentose phosphate pathway, amino acid metabolism, amino acid degradation, formation of DNA/RNA, protein, lipids, carbohydrates, glycosylation, respiration, and transport steps between intracellular compartments as well as between the cytosol and the extracellular environment.

The construction of a metabolic network model may include both the assembly of the available reactions into a mathematical representation by a stoichiometric matrix, as well as the verification of functionalities of pathways that are expected to be present in the cell line according to a priori biological knowledge. Genome sequence forms the basis of biochemical information used to reconstruct the network. Publicly available databases like KEGG (Kyoto Encyclopedia of Genes and Genomes) and Biocyc bridge the gap between genome sequence and biochemical reactions using genome annotation.

A metabolic network model may include:

a) Elemental compositions and molar masses of the metabolites.
b) A stoichiometric matrix containing stoichiometric coefficients which indicate the involvement and role (reactant / product) of various metabolites in the chemical reactions of the metabolic network.
c) Reversibility indices pointing out whether a reaction is reversible or not.
d) Assignment of enzymes that catalyze the individual reaction steps.

The stoichiometric matrix may be considered the central part of a metabolic network model, with the stoichiometric matrix being a m $\times$ n matrix including the stoichiometric coefficients of m metabolites participating in $n$ reactions. Each entry of the matrix is a stoichiometric coefficient $S_{i,j}$ which associates the metabolite $i$ to the reaction j where:

$$S_{i,j} = \begin{cases} S_{i,j} > 0, & \text{if metabolite } i \text{ is produced in reaction } j \\ S_{i,j} = 0, & \text{if metabolite } i \text{ is not involved in reaction } j \\ S_{i,j} < 0, & \text{if metabolite } i \text{ is consumed in reaction } j \end{cases}$$

The stoichiometric matrix is invariant and usually sparse since most biochemical reactions involve only a few different metabolites. In each row of a stoichiometric matrix a metabolite is balanced, and in every column a chemical reaction is elementally balanced.

A reaction is defined as irreversible, if the reaction only proceeds in one direction, and reversible, if the reverse reaction can also take place. For defining a metabolic model, reversibilities R may be defined as a $n$-dimensional vector of Boolean values for all reactions $n$ of the metabolic model.

$$R_j = \begin{cases} true, & \text{if reaction } j \text{ is reversible} \\ false, & \text{if reaction } j \text{ is irreversible} \end{cases}$$

A technique that is commonly used in connection with metabolic network models is Flux balance analysis. Flux balance analysis (FBA) is a mathematical approach for analyzing the reaction flux distribution of a metabolic network model. It may be used to calculate the flux distribution based on a constrained linear optimization problem. It is thereby possible to optimize (i.e., improve) the flux through a preferred metabolic reaction subject to a set of constraints on the metabolic network model. Thus, one can maximize (i.e., improve) the biomass formation or the production of a biotechnological target product by optimizing (i.e., improving) the metabolic reactions associated with the biomass formation or the product formation, respectively.

The linear optimization problem that is solved by FBA may provide an improved or optimal specific flux distribution of a metabolic network, which maximizes (i.e., increases) the desired performance index/target $Z = c^T v$ subject to the steady state constraints ($Sv = 0$) and lower/upper bounds $lb, ub$ on the flux vector $v$ as well as equality and inequality constraints $g$ and h.

Mathematically, the overall problem can be formulated as follows:

$$\max_{v} \quad Z = c^T v$$
$$\text{subject to} \quad Sv = 0$$
$$lb \leq v \leq ub$$
$$g \leq 0$$
$$h = 0$$

where

$Z = c^T v$    is the objective function

c    is the vector of coefficients, representing the weights of the reactions contributing to the objective function

$S$    is the stoichiometric matrix

v    is the flux vector describing all fluxes of a metabolic network in e.g., mmol /(gDW h) where gDW referes to gram dry weight.

$lb$    is a vector of lower bounds containing the fluxes

ub    is a vector of upper bounds containing the fluxes

g    is a vector of inequality constraints specific to the metabolic network model

$h$    is a vector of equality constraints specific to the metabolic network model

The aim of the FBA is maximization or minimization (i.e., an increase or decrease) of an objective function Z, thereby finding a unique solution in the allowed solution space, subject to a set of constraints. The objective function Z indicates which reactions may be maximized (i.e., increased) (positive weights c) or minimized (i.e., decreased) (negative weights c) by performing the FBA. The vector c (which is a vector of weights) is filled with zeros corresponding to the reactions of no interest and ones corresponding to the reactions of interest. For example, if the production rate of a specific target product is of interest, the vector c has the entry one for this specific reaction and is filled with zeros for all other reactions.

$$Z = c^T v$$

The general mass balance for a bioprocess without reaction can be written as follows:

$$Input = Output + Accumulation$$

However, since FBA assumes a mass balance at steady state, it implies that the accumulation term becomes zero. Mathematically, this can be expressed in matrix form as follows:

$$Sv = 0$$

where S represents the stoichiometric matrix obtained from the metabolic network model as a core, and $v$ refers to the flux vector which describes the fluxes of all the reactions of the metabolic network model. Usually, the number of reactions is higher than the number of metabolites associated with a metabolic network model. This means that the number of unknown variables exceeds the number of equations and hence, no unique solution exists for such a system.

Flux balance constraints narrow down the possible solution space of the above equation. They can be represented by either equations or inequalities. Equations define the reaction input or output fluxes exactly by a number, whereas inequalities just impose boundaries on the solution space. These constraints can be set by modifying the upper and lower bounds of the fluxes.

$$lb \leq v \leq ub$$

Metabolic Flux Analysis (MFA) is another technique commonly used with metabolic network models. MFA is a constrained least-squares minimization problem. It minimizes (i.e., decreases) the weighted mean squared error between resulting flux distributions and given observed fluxes while respecting various additional limitations. This weighted mean squared error objective function is mathematically represented by Z as:

$$\min_{e} \quad Z = (e - \hat{e})^T \cdot \frac{1}{W} \cdot (e - \hat{e})$$

$$\text{subject to} \quad \begin{aligned} e &\subseteq v \\ Sv &= 0 \\ lb \leq v &\leq ub \\ g &\leq 0 \\ h &= 0 \end{aligned}$$

where

Z is the objective function
v is the flux vector describing all fluxes of a metabolic network in e.g., mmol /(gDW h)
e is a vector of the resulting fluxes from MFA
$\hat{e}$ is a vector of the observed fluxes
W is a diagonal matrix containing the standard deviations of $\hat{e}$
S is the stoichiometric matrix
lb is a vector of lower bounds containing the fluxes
ub is a vector of upper bounds containing the fluxes
g is a vector of inequality constraints specific to the metabolic network model
h is a vector of equality constraints specific to the metabolic network model

Unlike an ordinary least square error term, the weighted least squares error does not assume a uniform standard deviation in all the measurements. A more precise measurement has a smaller standard deviation value and thereby a greater weight value (note that weights are calculated as element-wise inversion of $W$. This is represented as $\frac{1}{W}$ in the above equation). Such a greater weight value forces the algorithm to preferably fit the more precise measurements i.e., those with lower standard deviations. If a measurement is imprecise, it will have a corresponding greater standard deviation value and thereby a smaller weight value. This way the algorithm is more unlikely to fit such imprecise measurements. All in all, the weighted least squared error may allow the MFA algorithm to produce more reliable results when faced with non-uniformly distributed standard deviations.

Including the weighting factor in the objective function makes the algorithm capable of fitting the fluxes which are in different orders of magnitude (for example the consumption rate of a small molecule, e.g., glucose, is much larger than the production rate of a large molecule, e.g., glycoprotein). To perform the MFA algorithm, the simplex method from the CPLEX API (an API (Application Programming Interface) for solving optimization problems) may be used, for example.

Elementary Flux Mode Analysis (EFMA) is another technique commonly used with metabolic network models. A Flux Decomposition algorithm may decompose metabolic flux distributions into elementary flux modes (EFMs), which may be used to train the at least one machine-learning model of a Digital Twin (DT), as proposed in the present disclosure. EFMs are generally considered the smallest functional units in a metabolic network at steady state. This means that one cannot delete any reaction from an EFM and still obtain a valid steady-state flux distribution. Using distinctive sets of EFMs, one may be able to describe all possible flux distributions in a metabolic network model. Therefore, EFMs can mathematically characterize a metabolic network structure.

To obtain the EFMs using the Flux Decomposition algorithm, a metabolic network model consisting of a stoichiometric matrix S and a reversibilities vector R may be used as well as a set of flux distributions (e.g., obtained via Metabolic Flux Analysis (MFA) and/or (subsequent) Flux balance analysis (FBA)). The Flux Decomposition algorithm being used in the present disclosure is based on the work of Chan et al. ("Chan, S.H.J., and Ji, P. (2011). Decomposing flux distributions into elementary flux modes in genome-scale metabolic networks. Bioinformatics 27, 2256-2262.), with some modifications applied.

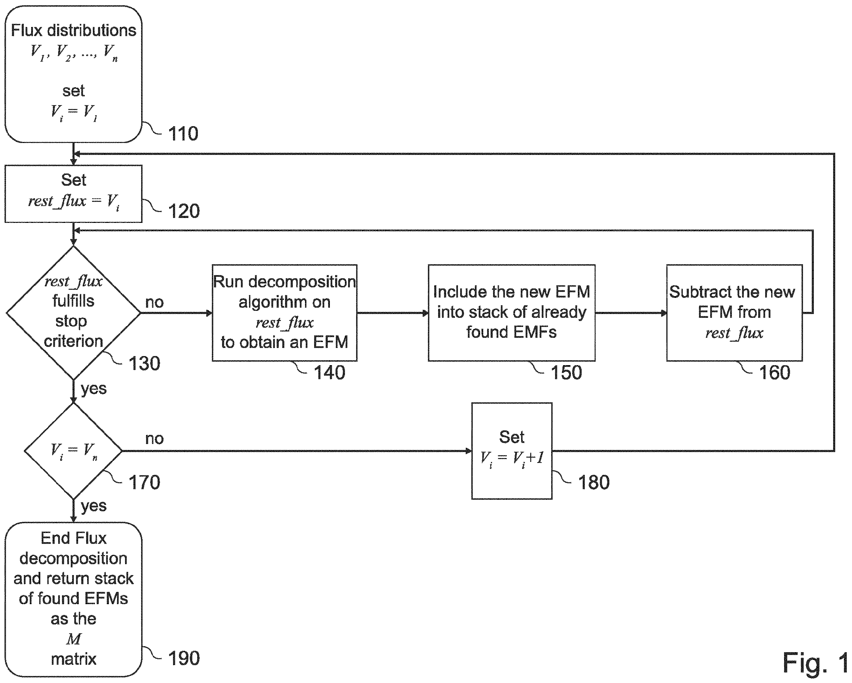

Fig. 1 shows a schematic representation of a flux decomposition algorithm according to an example. Initial conditions 110, flux distributions $V_1, V_2, ..., V_n$ set $V_i = V_n$. At the beginning, a variable called *rest_flux* may be initialized 120 with $V_i$. The Flux Decomposition algorithm may be performed 140 on *rest_flux* to obtain an EFM. Once an EFM is found, it may be added 150 to the stack of found EFMs and subtracted 160 from the *rest_flux.* This procedure may be repeated until *rest_flux* fulfills the certain stop criterion (see section 2.4) 130. If this is the case, the Flux Decomposition for $V_i$ may end and the same procedure may be performed for $V_{i+1}$ 180. This loop may continue until the last flux distribution i.e., $V_n$ is completely decomposed 170. Finally, the full stack of all found EFMs is the result of the Flux Decomposition algorithm and may be saved in the mode matrix M 190. A possible prerequisite of the Flux Decomposition algorithm is converting all reversible reactions into an irreversible form. This is explained in detail in the following.

To perform the Flux Decomposition, the reactions in the metabolic network model may be converted into an irreversible form. Reversible reactions may be split into two irreversible reactions (forward $j^+$ and backward $j^-$) with stoichiometric coefficient and flux values with opposite signs. This can be expressed using the following equation:

$$R_{j+} = 0$$

$$R_{j-} = 0$$

$$S_{i,j^-} = -S_{i,j^+} = -S_{i,j}$$

$$v_{j^-}^{c,t} = -v_{j^+}^{c,t} = -v_j^{c,t} \quad \forall i \in I, \forall j: \{j \in J \mid R_j = 1\}, \forall v^{c,t}: \{c \in C, t \in T\}$$

where

$i \in I$    is a metabolic compound (e.g., ATP, Adenosine triphosphate)
$j \in J$    is a reaction (e.g., atpase1)
$c \in C$    is a cultivation process (e.g., cultivation1)
$t \in T$    is a time point during cultivation (e.g., 12.0 [h])
$R_j$    is the reversibility vector entry of the reaction j
$R_j+$    is the reversibility vector entry of the irreversible forward reaction form of reaction j
$R_j-$    is the reversibility vector entry of the irreversible backward reaction form of reaction j
$S_{i,j}$    is the stoichiometric matrix entry for the compound *i* and the reaction j
$S_{i,j}+$    is the stoichiometric matrix entry for the compound *i* and the forward reaction j
$S_{i,j}-$    is the stoichiometric matrix entry for the compound *i* and the backward reaction j

$v_j^{c,t}$        is the FBA flux for the reaction *j,* for cultivation c at time *t*

$v_{j+}^{c,t}$        is the FBA flux for the forward reaction *j,* for cultivation c at time *t*

$v_{j-}^{c,t}$        is the FBA flux for the backward reaction *j,* for cultivation c at time *t*

To convert the reversible reactions into an irreversible form, the next task may be to identify the EFMs in the metabolic network model. In general, to define *p* as an EFM bounded by the flux distribution *v*, the following five different constraints may be taken into account:

1. An elementary flux mode *p* must satisfy the steady-state condition

$$Sp = 0$$

where S is the stoichiometric matrix.

2. For each reaction $j$, there is a binary integer variable $a_j$ which determines whether the reaction $j$ is ON (i.e., $a_j = 1$) or OFF (i.e., $a_j = 0$). The binary variable $a_j$ must satisfy the inequality term

$$0.1a_j \leq p_j \leq Q\delta_j a_j$$

where $Q$ is a large positive number and $\delta_j$ is also a binary variable which indicates the activity function of the flux which is going to be decomposed. $\delta_j = 1$ if $v_j > 0$, otherwise $\delta_j = 0$ ($v_j$ indicates the flux of reaction j). According to the second constraint, if $a_j = 0$ (i.e., the reaction $j$ is OFF) then $p_j = 0$ and thereby the reaction $j$ is not involved. However, if $a_j = 1$ (i.e., the reaction j is ON) then $p_j \geq 0.1$ and thereby the reaction j must have a positive flux. The binary variable $\delta_j$ is a unique feature of the second constraint meaning that if $\delta_j = 1$ then $p_j$ and $a_j$ are forced to be zero. This, in other words, means that if $v_j = 0$ then $p_j = 0$. The variable $Q$ should be large enough to allow all flux modes bounded by $v$ to satisfy the above constraint. $Q$ can be interpreted as the greatest stoichiometric ratio allowed within the flux mode $p$.

3. To make sure that $p$ is non-trivial (i.e., it has at least one non-zero flux):

$$\sum_i a_j \geq 1$$

4. As mentioned earlier, the elementary flux mode $p$ must be bounded by $v$. To ensure this, the number of non-zero fluxes in $p$ must be less than the number of non-zero fluxes in $v$:

$$\sum_j a_j \leq \sum_j \delta_j - 1$$

5. The fifth constraint takes care that all fluxes must be positive. This should be the case as all reactions are irreversible:

$$p_j \geq 0$$

The above constraints may be sufficient to make sure that the elementary flux mode $p$ is bounded by $v$. Thereby, every objective function may work so the objective function is set to *max*( ). The improvement or optimization algorithm aims at maximizing (i.e., increasing) the number of zero $a_j$ values. This may enforce the sparseness of the algorithm such that the system can be described with a minimum (or reduced) set of non-zero fluxes. The five constraints above, as well as the objective function of the optimization, may construct a mixed integer linear problem. If for a certain elementary flux mode $v$ any feasible solution is found, then v is still decomposable, otherwise $v$ may be considered an EFM.
After a new EFM is identified, it may be added to the stack of already found modes. In an optimization (i.e., improvement) problem these modes are subtracted from the current rest flux by maximizing (i.e., increasing) the coefficients of their positive linear combinations.

$$
\begin{aligned}
max \quad & (r) \\
\text{s.t.} \quad & v_j^{rest*} + \sum_{z=1}^{m^{mod}} r_z \cdot M_{j,z} = v_j^{rest} \\
& S \cdot v^{rest*} = 0 \qquad \forall j \in E, M \in \mathbb{R}^{n \times m^{mod}}, S \in \mathbb{R}^{m \times n}, r \in \mathbb{R}^{m^{mod}} \\
& r \geq 0
\end{aligned}
$$

wh ere

$j \in E$     denotes only the observed exchange reactions
m        is the number of metabolites in the stoichiometric matrix S
$n$        is the number of reactions (transformers) in the stoichiometric matrix S

S is the stoichiometric matrix

$m^{mod}$ is the number of found elementary flux modes

M is the matrix of already found elementary flux modes from the stack

$v^{rest}$ is the rest flux at the current iteration

r is a weight vector with the coefficients of the linear combination of elementary modes from the modes matrix M to describe $v^{rest}$

$v^{rest*}$ is the residual between rest flux and the linear combination of EFMs

To stop the algorithm from explaining even small rest fluxes $v_{n+1}^{rest}$ caused by noise, a stop criterion may be defined. The stop criterion $\varepsilon$ may be calculated as residual between rest flux and the linear combination of EFMs $v^{rest*}$ divided by the standard deviations $\sigma$ of the initial FBA flux distribution $v$, squared and meaned (weighted mean square error, WMSE):

$$\epsilon = \sum_{j=1}^{e} \frac{1}{e} \left( \frac{v_j^{rest*}}{\sigma_j} \right)^2 \quad j \in E$$

where

$\varepsilon$ is the stop criterion

$v_j^{rest*}$ is the residual between rest flux and the linear combination of EFMs for exchange reaction $j \in E$

$\sigma_j$ is the corresponding standard deviation of the initial FBA flux distribution for exchange reaction $j \in E$

$e$ is the number of exchange reactions.

To make the results comparable for exchange fluxes of metabolites with very molar masses (i.e., $CO_2$ vs. Biomass or Product), the flux may be multiplied for each exchange flux with its respective molar mass (molecular weight $w_j$):

$$\tilde{v}_j^* = \sum_{z=1}^{m^{mod}} r_z \cdot M_{j,z} \cdot w_j \quad j \in E$$

$$\tilde{v}_j = v_j \cdot w_j \qquad j \in E$$

where

$v_j$ is the initial FBA flux distribution for the observed exchange flux $j \in E$

$w_j$ is the corresponding molecular weight for the metabolite of the observed exchange flux $j \in E$

$\tilde{v}_J$ is the initial FBA flux distribution for the observed exchange flux $j \in E$ weighted with the corresponding molecular weight

$m^{mod}$ is the number of found elementary flux modes

M is the matrix of already found elementary flux modes from the stack

r is a weight vector with the coefficients of the linear combination of elementary modes from the modes matrix M

$\widetilde{v_j^*}$ is the explained flux distribution in terms of maximal positive linear combinations of EFMs weighted with the molar masses

If the stop criterion is reached, the remaining flux $v^{rest*}$ might not further be decomposed and a new FBA flux from the FBA stack may be used to find new EFMs. The stop criterion is a suitable tool to define the number of EFMs found by the Flux Decomposition. Per default it may be set to a pre-defined low value, such as 0.0001.

The result of the Flux Decomposition may be an elementary flux modes matrix M, which includes (all of) the identified EFMs. The dimensions of M are defined by the number of reactions (transformers) of the metabolic network model and the number of identified EFMs.

In various examples of the present disclosure, a concept called Metabolic Base Functionalities (MBF), also known as Base Nodes, may be used. The Digital Twin may learn the impact of its inputs (e.g., representations of an environment of the biological organism, such as one or more of the following: concentrations, reactor volume, feeding, sampling, additional inputs such as pH, temperature, and expression levels) on metabolic rates by means of elementary flux modes (EFMs) activities. In general, EFMs may be obtained via EFMA, which may use the observed exchange fluxes by Metabolic Flux Analysis (MFA) and subsequent Flux balance analysis (FBA) on estimated exchange rates. In other approaches, this workflow may be run from scratch for each new project or data which is cost intensive. Additionally, the quality of estimated rates might suffer due to limited data availability and measurement noise.

It is a finding of the proposed concept, that a large number of metabolic functionalities are common across projects for the same organism or cell line. Examples may include biomass and product formation, production of non-essential amino acids, as well as degradation of certain metabolites. As part of the generation of metabolic network models, certain (required) metabolic functions may already be defined, which are a subset of these common functionalities. They may be derived via targeted Flux balance analysis (FBA) and can be further extended by applying expert knowledge. One example is the conversion of amino acids into another. These base metabolic functionalities may be appended to EFMs or can be used as a starting point for a subsequent EFMA and reduce the number of required EFMs, which may decrease the computation time of the flux decomposition algorithm or meet the challenge of reduced data. In the Digital Twin, EFMs as well as metabolic base functionalities can be re-used across projects.

For example, the aforementioned metabolic base functionalities may be obtained via targeted Flux balance analysis (FBA), e.g., by means of setting constraints in the following way: The exchange fluxes of expected substrates may be constrained to be negative and of product positive (often also fixating the main substrate to a constant value to meet the unboundedness problem), and the other exchange fluxes may be constrained to zero. Additionally, known yields may be fixated, for example known substrate demands for product formation.

For example, for the metabolic base functionality "glucose degradation" the exchange flux for glucose may be set to -1 and the ones for $CO_2$, $H_2O$, and H may be left unconstrained while constraining all other exchange fluxes to be zero.

Optionally, Flux Decomposition can be run to complement missing functionalities.

In the following, an example is given for a reactor model of the bioreactor (hosting the biological organism). The reactor model may represent (any) concentration and volume changes in the bioreactor due to influx (i.e., feeding) and outflux (e.g., sampling, permeate outflux with cell retention and bleed flux without cell retention in perfusion processes). The change in volume $V(t)$ can be described using the following differential equation:

$$\left.\frac{dV}{dt}\right|_{reactor} = \sum_{i=1}^{q} F_i^I(t) - \sum_{j=1}^{p} F_j^O(t) - E(t)$$

$F_i^I(t)$ represents the addition of volume due to feeding from the medium $i$. $F^I(t)$ is considered to be the row-vector of all feeding rates. $F_j^O(t)$ is the outflux rate, which can be defined as the reduction of volume due to any continuous extractions of media from the bioreactor. $F^O(t)$ is considered to be the row-vector of all outflux rates.

The biomass concentration $X(t)$ changes due to outflux of cells relative to the existing biomass concentration.

$$\left.\frac{dX}{dt}\right|_{reactor} = -\sum_{j=1}^{p} \left( \frac{F_j^O}{V(t)} \cdot \left(1 - R_{j,X}\right) \cdot X(t) \right)$$

Each outflux $j$ has its own cell retention factor $R_{j,X}$ which indicates how much of the biomass is held back. This means that a cell retention factor of 0 corresponds to no retention at all whereas a cell retention factor of 1 corresponds to total retention. Cell retention factors are considered to be constant within one timestep.

The concentrations C(t) of non-biomass metabolites change due to feeding and outflux with possible retention given by factor $R_j$:

$$\frac{dC}{dt}\bigg|_{reactor} = \sum_{i=1}^{q}\left(\frac{F_i^I}{V(t)}\cdot C_i^I\right) - \sum_{j=1}^{p}\left(\frac{F_j^O}{V(t)}\cdot\left(1-R_j\right)\cdot C(t)\right)$$

where $C_i^I$ is the vector of metabolite concentrations in feed $i$.

In addition to the reactor model, the Digital Twin may use an extracellular reaction model. Extracellular reactions are typically chemical degradation reactions taking place in the cultivation medium independent of the biomass concentration. One example of such reactions is degradation of glutamine to 5-oxoproline and ammonia. The change in the different concentrations may be modeled using mass-action kinetics. In general, the abiotic degradation reaction of substrate S into product P with the rate $k_{deg}$ can mathematically be shown as:

$$\frac{dC_S}{dt}\bigg|_{extracellular} = -k_{deg}\cdot C_S(t)$$

$$\frac{dC_P}{dt}\bigg|_{extracellular} = k_{deg}\cdot C_S(t)$$

As another component, the Digital Twin may comprise a generic osmolality model. The osmolality of a solution is the number of particles of solute dissolved in one kilogram of the given solution. In pharmaceutical industries, osmolality is an important control and quality-check parameter throughout the development and manufacture of biologics. Monitoring the osmolality value helps to ensure cell health and to reduce the risk of process failure. Thereby, the osmolality may be taken into account when training a Digital Twin (DT), and in particular the at least one machine-learning model of the DT. To do so, an osmolality model (or submodel) may be trained and incorporated in the Digital Twin. Such an osmolality model can predict the experimentally measured osmolality values. This makes it possible to control the osmolality of the processes being improved optimized by the DT. This way, the cell health in optimized processes may be supported ensured and the risk of a process failure may be reduced.

For example, the osmolality model may be a linear regression model which is mathematically described as:

$$y^N(t) = \sum_i \alpha_i \cdot c_i^N(t) + \beta$$

where:

$y^N(t)$ is the normalized experimentally measured osmolality value (i.e., the term $N$ stands for normalized) at time point $t$. The normalization is of type max-normalization

$\alpha_i$ is the coefficient corresponding to the metabolite $i$

$c_i^N(t)$ is the normalized experimentally measured concentration of metabolite $i$ at time point $t$ in the bioreactor. The normalization is of type metabolite-wise max-normalization. Biomass, dead-biomass, and glycoprotein are excluded.

$\beta$ is the intercept

The linear regression model may be of type "Lasso", which may be trained based on L1 regularization. The algorithm may take the normalized experimentally measured concentration of compounds (i.e., $c_i^N(t)$ ) and predict the normalized experimentally measured osmolality values (i.e., $y^N(t)$), at time point $t$. This may be done by estimating the best $\alpha_i$ values as well as the best $\beta$ value.

In many cases, osmolality of a solution may be calculated as weighted sum of the concentration of compounds in the solution, considering the weights being positive. Therefore, in the Lasso algorithm $\alpha_i$ values (which are so called weights or coefficients here) are forced to be positive. Once normalized osmolality values i.e., $y^N(t)$ are predicted, one can apply de-normalization and derive the actual estimated osmolality values.

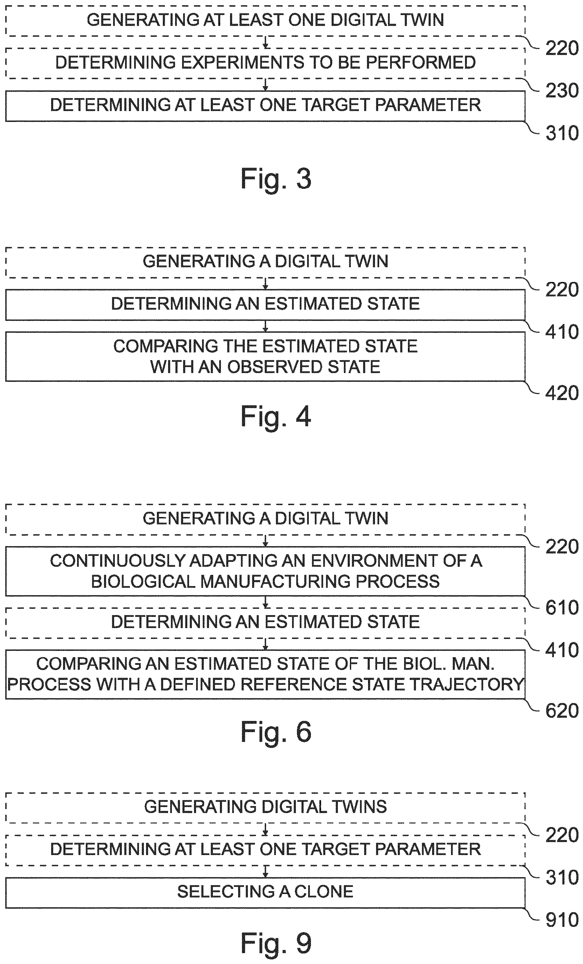

The trained osmolality model may be used at later stages during process improvement or optimization (see e.g., Fig. 9) in which concentration of compounds in the media and/or the feeding strategies are improved or optimized, respectively. Media and/or feed optimization may lead to different simulated time-resolved bioreactor concentration profiles than the experimentally measured values. Having $\alpha_i$ and $\beta$ estimated, using the above equation, one can estimate time-resolved osmolality values corresponding to the optimized process(es). To do so, the improved or optimized time-resolved bioreactor concentration profiles i.e., $c_i^N(t)$ may be used in the above equation to estimate the corresponding osmolality values i.e., $y^N(t)$. Such normalized osmolality values may be de-normalized and thereby actual time-resolved osmolality values can be estimated. This helps to keep the osmolality of the improved or optimized process(es) in a certain (reasonable) range such that the cell health is supported or ensured, and the improved or optimized process(es) are at lower risk of failure.

Practically, bioreactor concentration measurements may not be available for each individual time point. If measured concentration of a certain metabolite at a certain time point is missing, the $c_i^N(t)$ is incomplete (i.e., $c_i^N(t) = NaN$ for the metabolite $i$ at time point t). Therefore, the corresponding osmolality value at this specific time point might not be predicted.

To resolve this issue, a down-sampling approach may be applied. Using the down-sampling approach, time resolution of the data is set to a high value (e.g., $\Delta t = 24h$). Then, the average of all measured concentrations values between $t$ and $t + \Delta t$ may be calculated and set as the measured concentration at time point $t + \Delta t$. The same method may also be applied on the osmolality values measured between $t$ and $t + \Delta t$. This way, even if one or more concentration measurements are not available in the time period between $t$ and $t + \Delta t$, it would still be possible to have a value (i.e., the down-sampled one) available at $t + \Delta t$. This way, the chance that $c_i^N(t)$ is complete for more time points increases, and thereby more data points are used to train the osmolality value. The more available time points in the data, the better the trained osmolality model usually is.

In case for a certain metabolite, the concentration measurement exists for less than 50% of all the time points (across all processes), this metabolite may be discarded from the osmolality prediction procedure.

Some examples of the present disclosure relate to product quality attributes (PQAs). Product quality attributes such as glycosylation and charge variants of the product are modelled in terms of percentages of the total product titer:

$$C_{var\,j} = \frac{p_{var\,j,\%} \cdot C_P}{100}$$

where:

$C_{var\,j}$ is the concentration of the PQA $j$
$p_{var\,j,\%}$ is the percentage value of the contribution of the *PQA j* to the total product titer
$C_P$ is the product concentration (titer)

In the following, a so-called generalized kinetic cell model (GKCM), a generic modes matrix, a clone-specific mode matrix and generic and clone-specific parameters are being discussed. These may be used, in combination, to model the kinetic aspects of the biological organism.

The generic and clone-specific parameters may correspond to components of at least one machine-learning models, e.g., to layers of one or multiple artificial neural networks, or to different machine-learning models. In the following, the application of the GKCM, comprising the generic and clone-specific parameters, are introduced, followed by the training.

The generalized kinetic cell model (GKCM) simulates concentration changes caused by cell metabolism using differential equations. A differential equation that can be used for biomass concentration $X(t)$ is:

$$\left.\frac{dX}{dt}\right|_{cell} = \hat{\mu}(t) \cdot X(t) - k_{death} \cdot X(t)$$

As indicated in the above equation, biomass concentration may depend on the current biomass concentration, the biomass growth rate (often referred to only as growth rate) $\hat{\mu}(t)$ and cell death described by the linear constant $k_{death}$. In the following examples, $\mu(t) = \hat{\mu}(t) - k_{death}$ to describe the growth ($\mu(t) > 0$) and death ($\mu(t) < 0$) of biomass with a single rate.

For the metabolite concentrations, the equation may differ as the change of metabolite concentrations may depends on the biomass concentration with the biomass-specific consumption/production rate r(t). This may be mathematically represented by the following differential equation:

$$\left.\frac{dC}{dt}\right|_{cell} = r(t) \cdot X(t)$$

The growth rate and the biomass specific consumption/production rates may depend on the current biomass and metabolite concentrations. The variable C(t) here refers to the vector of all concentrations $c_i(t)$. The rates $\mu(t)$, r(t) are possible outputs of the kinetics given by the machine-learning model(s) (e.g., the neural networks, see below for the different architecture types) and the stoichiometry (given by EFMs and/or the metabolic base functionalities). A general mathematical description may be given by:

$$[\mu(t), r(t)]^T = f^{kinetic}\left(M^{generic}, M^{clone-specific}, t, x^{envirome}(t), \theta^{generic}, \theta^{clone-specific}\right)$$

where

| | |
|---|---|
| $f^{kinetic}(\cdot)$ | is the kinetic relation trained in the neural networks combined with the stoichiometry from the metabolic network model |
| $M^{generic}$ | is the matrix containing elementary flux modes (EFMs) and/or metabolic base functionalities with defined order. In particular, the function and order of modes is the same in each clone (hence generic). Only the biomass and product synthesis modes may differ across clones due to differences in the biomass or product composition |
| $M^{clone-specific}$ | is the matrix containing a clone specific number of elementary flux modes (EFMs) and/or metabolic base functionalities with clone-specific functionality |
| $x^{envirome}(t)$ | $[X(t), c(t), p(t)]^T$ |
| $p(t)$ | is the vector of additional parameters such as pH and temperature that can be used as additional input for the kinetic cell model |
| $\theta^{generic}$ | is the set of generic kinetic cell model parameters which can be trained on data from a large number of clones and shared across clones |
| $\theta^{clone-specific}$ | is the set of clone-specific kinetic cell model parameters which are only trained on data from a particular clone |

The generic modes matrix $M^{generic}$ may contain elementary flux modes or base metabolic functions with defined functionality and order. In particular, the function and order of modes may be the same in each clone. Only the fluxes within the biomass and product synthesis modes might differ across clones due to differences in the biomass or product composition. Despite differences in individual fluxes in those modes, these modes may have the same functionality and are located at the same place in the matrix across clones. As a consequence, a generic kinetic model may be trained for the functionality they represent.

The clone-specific modes matrix $M^{clone-specific}$ may contain an arbitrary, i.e., clone-specific number of modes with arbitrary, i.e., clone-specific functionality

In the following, the generic and clone-specific parameters are discussed. They may correspond to a first and a second component of the at least one machine-learning model used for modeling the kinetics of the biological organism.

$\theta^{generic}$ (e.g., which may form, as $\theta^{generic}_{1,3}$, the first and/or third component of the at least one machine-learning model) is a set of parameters that is calibrated using data from (all of) the available processes in the GKCM training set (e.g., from the entire training data). $\theta^{clone-specific}$ (e.g., which may form, as $\theta^{clone-specific}_{2,4}$, the second and/or fourth component of the at least one machine-learning model) is a different set of parameters that may exist for each clone in the training data and can be different for each clone, as it is only calibrated using the training data corresponding to the respective clone (e.g., a subset of training data that is based on experimental data of a single clone).

In some cases, especially when initially generating a GKCM, $\theta^{generic}$ and $\theta^{clone-specific}$ may be trained in parallel on a large number of data sets from different clones. In other cases, e.g., when a GKCM was previously trained on historical data and only a reduced data set for a new clone is available (without the historical data), warm starts may be applied, i.e., $\theta^{generic}$ may be loaded from the previously trained GKCM and remain unchanged while the clone specific

parameters $\theta^{clone\text{-}specific}$ corresponding to the new clone are calibrated on the new data.

Optionally, generic compression may be used to deal with measurement vectors of different sizes. In general, it may be recommended to use a low-dimensional representation of the input vector (which may represent the environment of the biological organism) which may be comprised of (all of) the factors that can affect the state of the organism. The input vector may be defined as

$$x^{envirome} = [X, c, p]^T$$

Such a low-dimensional representation (which may correspond to a compressed representation of the environment) can for instance be achieved with linear methods (like PCA) or with the compressing part of an autoencoder. In general, the mapping function may be defined as

$$\hat{x}^{envirome} = f^{compression}\left(x^{envirome}, \theta^{compression}\right)$$

where $\theta^{compression}$ is a subset of $\theta^{generic}$.

In various examples, some general constraints may be applied to $f^{kinetic}$. The sum of fluxes through any mode may (have to) be positive in rate equation $f^{kinetic}$. Negative mode fluxes may revert fluxes of irreversible reaction and hence lead to thermodynamically infeasible fluxes. Furthermore, in the above notation, mode matrices may contain exchange reactions (which uniquely map to extracellular compounds with factor 1), i.e., stoichiometric mapping to extracellular compounds may be contained. Otherwise, a matrix multiplication of the mode matrix with the stoichiometric matrix may be used to map mode fluxes to growth and exchange rates.

In some examples, regularization may be used. In the context of training a GKCM, it may be recommended to particularly regularize the clone-specific parts (e.g., fluxes of $M^{clone\text{-}specific}$ and $\theta^{clone\text{-}specific}$) Concrete examples for $f^{kinetic}$

$$f^{kinetic} = f^{kinetic}\left(M^{generic}, M^{clone-specific}, t, x^{envirome}(t), \theta^{generic}, \theta^{clone-specific}\right)$$

$$= M^{generic} \cdot f_3\left(f_1\left(x^{envirome}(t), \theta^{compression}, \theta_1^{generic}\right),\right.$$

$$f_2\left(x^{envirome}(t), \theta^{compression}, \theta_2^{clone-specific}\right), \theta_3^{generic}\right) + M^{clone-specific}$$

$$\left. \cdot f_4\left(x^{envirome}(t), \theta^{compression}, \theta_4^{clone-specific}\right)\right)$$

In the following, the term $ANN_i$ is used to describe different artificial neural networks, e.g., feed forward neural networks, as function of the specified inputs. As the output of these neural networks is often limited, e.g., between 0 and 1, it is in general multiplied with a positive scalar, vector, or matrix, which is denoted here as $H_i$.

The following examples are given with respect to ANNs (Artificial Neural Networks) as a specific example of machine-learning models. However, other types of machine-learning models may be used as well.

In the following, two examples of architectures for combining the generic and clone-specific parts of the GKCM are given. As will become evident later, depending on the architecture chosen, the training of the at least one machine-learning model may be suitably adapted. Moreover, the proposed concept is not limited to the two architectures given below.

In a first architecture example, multiplication is used to combine the generic and clone-specific parts of the GKCM. The generic part $f_1$ and the clone-specific part $f_2$ may be combined using the following architecture

$$f_3\left(f_1, f_2, \theta_3^{generic}\right)$$

$$= f_1\left(x^{envirome}(t), \theta^{compression}, \theta_1^{generic}\right)$$

$$\cdot \left(1 + f_2\left(x^{envirome}(t), \theta^{compression}, \theta_2^{clone-specific}\right)\right) \cdot H_3,$$

$$\theta_3^{generic} = H_3 \geq 0,$$

where $f_1$ is

$$f_1\left(x^{envirome}(t), \theta^{compression}, \theta_1^{generic}\right) = ANN_1\left(\hat{x}^{envirome}(t), \theta_{ANN1}^{generic}\right) \cdot H_1,$$

$$ANN_1 \geq 0, H_1 \geq 0, \theta_1^{generic} = \{\theta_{ANN1}^{generic}, H_1\}, \theta_{ANN1}^{generic} = \{W_{ANN1}, b_{ANN1}\}$$

$f_2$ is

$$f_2\left(x^{envirome}(t), \theta^{compression}, \theta_2^{clone-specific}\right)$$

$$= max\left(-1, ANN_2\left(\hat{x}^{envirome}(t), \theta_{ANN2}^{clone-specific}\right) \cdot H_2\right),$$

$$H_2 \geq 0, \theta_2^{clone-specific} = \{\theta_{ANN2}^{clone-specific}, H_2\}, \theta_{ANN2}^{clone-specific} = \{W_{ANN2}, b_{ANN2}\}$$

and $f_4$ is

$$f_4\left(x^{envirome}(t), \theta^{compression}, \theta_4^{clone-specific}\right) = ANN_4\left(\hat{x}^{envirome}(t), \theta_{ANN4}^{clone-specific}\right) \cdot H_4,$$

$$ANN_4 \geq 0 \quad H_4 \geq 0, \theta_4^{clone-specific}$$

$$= \{\theta_{ANN4}^{clone-specific}, H_4\}, \theta_{ANN4}^{clone-specific} = \{W_{ANN4}, b_{ANN4}\}$$

In the first architecture, at least one machine-learning model with a first component $f1$ (comprising generic kinetic cell model parameters $\theta_1^{generic}$), a second component, $f_2$(comprising clone-specific kinetic cell model parameters $\theta_2^{clone-specific}$), and a fourth component $f_4$(comprising clone-specific kinetic cell model parameters $\theta_4^{clone-specific}$) is used.

In a second architecture, the first and second component of the at least one machine-learning model are combined using a third component of the at least one machine-learning model. In the second architecture, the generic part $f_1$ and the clone-specific part $f_2$ are combined using the following architecture for $f_3$:

$$f_3\left(f_1, f_2, \theta_3^{generic}\right)$$

$$= ANN_3\left(f_1\left(x^{envirome}(t), \theta^{compression}, \theta_1^{generic}\right),\right.$$

$$\left. f_2\left(x^{envirome}(t), \theta^{compression}, \theta_2^{clone-specific}\right), \theta_{ANN3}^{generic}\right) \cdot H_3,$$

$$ANN_3 \geq 0, H_3 \geq 0, \theta_3^{generic} = \{\theta_{ANN3}^{generic}, H_3\}, \theta_{ANN3}^{generic} = \{W_{ANN3}, b_{ANN3}\}$$

where $f_1$ is

$$f_1\left(x^{envirome}(t), \theta^{compression}, \theta_1^{generic}\right) = ANN_1\left(\hat{x}^{envirome}(t), \theta_{ANN1}^{generic}\right) \cdot H_1,$$

$$ANN_1 \geq 0 \; H_1 \geq 0, \theta_1^{generic} = \{\theta_{ANN1}^{generic}, H_1\}, \; \theta_{ANN1}^{generic} = \{W_{ANN1}, b_{ANN1}\}$$

and $f_2$ is

$$f_2\left(x^{envirome}(t), \theta^{compression}, \theta_2^{clone-specific}\right) = ANN_2\left(\hat{x}^{envirome}(t), \theta_{ANN2}^{clone-specific}\right) \cdot H_2,$$

$$H_2 \geq 0, \theta_2^{clone-specific} = \{\theta_{ANN2}^{clone-specific}, H_2\}, \; \theta_{ANN2}^{clone-specific} = \{W_{ANN2}, b_{ANN2}\}$$

and $f_4$ is

$$f_4\left(x^{envirome}(t), \theta^{compression}, \theta_4^{clone-specific}\right) = ANN_4\left(\hat{x}^{envirome}(t), \theta_{ANN4}^{clone-specific}\right) \cdot H_4,$$

$$ANN_4 \geq 0 \quad H_4 \geq 0, \theta_4^{clone-specific}$$

$$= \{\theta_{ANN4}^{clone-specific}, H_4\}, \theta_{ANN4}^{clone-specific} = \{W_{ANN4}, b_{ANN4}\}$$

In the second architecture, at least one machine-learning model with a first component $f_1$(comprising generic kinetic cell model parameters $\theta_1^{generic}$), a second component, $f_2$(comprising clone-specific kinetic cell model parameters $\theta_2^{clone-specific}$), a third component $f_3$(comprising generic kinetic cell model parameters $\theta_3^{generic}$), and a fourth component $f_4$(comprising clone-specific kinetic cell model parameters $\theta_4^{clone-specific}$) is used.

In both of the above examples, $\hat{x}^{envirome}(t)$ may result from different generic compressions depending on the available measurements for elements in $\hat{x}^{envirorne}(t)$:

$$\hat{x}^{envirome}(t) = f_i^{compression}\left(x_i^{envirome}(t), \theta_i^{compression}\right)$$

where $x_i^{envirome}$ includes the respective intersection of available measurements as a subset of $x^{envirome}$ in the data from all clones (input to generic part $f_1$) or in the data of the corresponding clone (input to clone-specific part $f_2$ and $f_4$) respectively. Note that in the simplest case, $f_i^{compression} = Identity$.

In some examples, data augmentation for interpretable generic behavior may be used. Data augmentation may be used to make the generic parts of the model more interpretable. Normally, process data is linked to a specific clone. However, process data can be augmented such that each process is duplicated leading to the original set of processes which are linked to specific clones and another set of processes which are not linked to specific clones. For processes not linked to a specific clone, the output of $f_2$ and $f_4$ may be 0. As a consequence, a generic behavior may be identified as output of $f_1$ and $f_3$ in combination with $f_2 = f_4 = 0$.

In the following, an example of a mathematical description of the Digital Twin is given. In various examples, the overall mathematical description of the Digital Twin is as follows:

$$\frac{dV}{dt} = \sum_{i=1}^{q} F_i^I(t) - \sum_{j=1}^{p} F_j^O(t) - E(t)$$

$$\frac{dX}{dt} = \left(\mu(t) - \sum_{j=1}^{p}\left(\frac{F_j^O}{V(t)} \cdot \left(1 - R_{j,X}\right)\right)\right) \cdot X(t)$$

$$\frac{dC_U}{dt} = r_U(t) \cdot X(t) + \sum_{i=1}^{q}\left(\frac{F_i^I}{V(t)} \cdot C_{U,i}^I\right) - \sum_{j=1}^{p}\left(\frac{F_j^O}{V(t)} \cdot \left(1 - R_{U,j}\right) \cdot C_{U(t)}\right)$$

$$\frac{dC_S}{dt} = r_S(t) \cdot X(t) + \sum_{i=1}^{q}\left(\frac{F_i^I}{V(t)} \cdot C_{S,i}^I\right) - \left(k_{deg} + \sum_{j=1}^{p}\left(\frac{F_j^O}{V(t)} \cdot \left(1 - R_{S,j}\right)\right)\right) \cdot C_S(t)$$

$$\frac{dC_P}{dt} = r_P(t) \cdot X(t) + \sum_{i=1}^{q}\left(\frac{F_i^I}{V(t)} \cdot C_{P,i}^I\right) - \sum_{j=1}^{p}\left(\frac{F_j^O}{V(t)} \cdot \left(1 - R_{P,j}\right) \cdot C_P(t)\right) + k_{deg} \cdot C_S(t)$$

where

U    is the index for metabolites not involved in extracellular reactions
S    is the index for substrates involved in extracellular reactions
P    is the index for products involved in extracellular reactions

and

$$[\mu(t), r(t)]^T =$$

$$f^{kinetic}\left(M^{generic}, M^{clone-specific}, t, x^{envirome}(t), \theta^{generic}, \theta^{clone-specific}\right).$$

In other words, the GKCM, and thus the at least one machine-learning model with the first, second, and optionally third and/or fourth, component may be used, to determine the ratees $[\mu(t),r(t)]^T$ as part of the Digital Twin.

The functionalities contained in the modes matrices may be metabolic base functionalities and/or EFMs derived from flux distributions from MFA on observed exchange rates that can be obtained via rate estimation (e.g., via a Digital Twin with a black-box (data-driven) kinetic cell model).

In the following, the training of the Digital Twin is discussed. This includes the training of the at least one machine-learning model, which may be used as part of the GKCM.

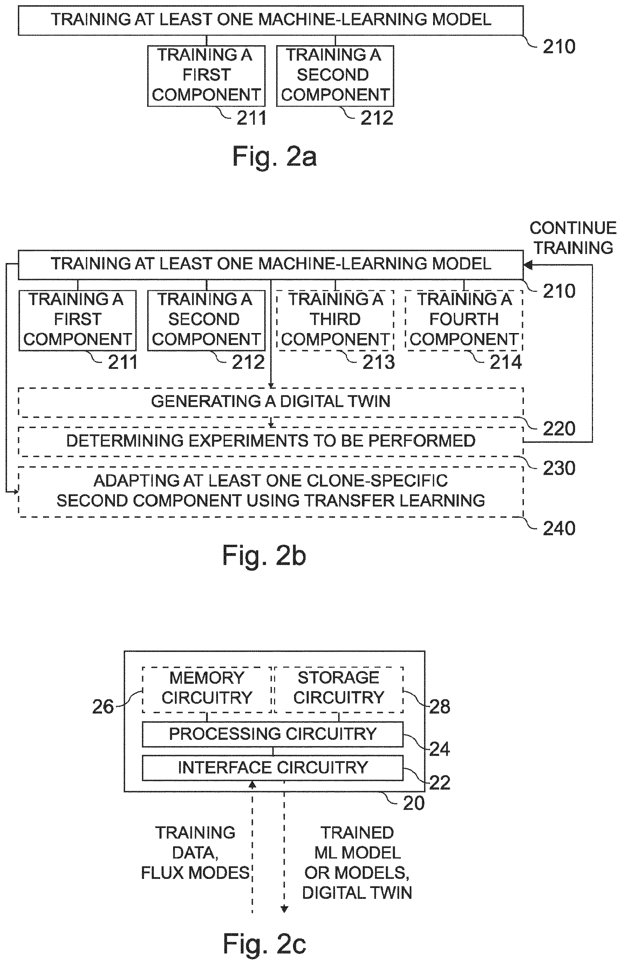

Figs. 2a and 2b show flow charts of examples of a method for training at least one machine-learning model for modelling kinetic aspects of a biological organism. The method comprises training 210 the machine-learning model based on training data. The training data is based on experimental data of a plurality of clones of the biological organism. The training data comprises a subset of training data that is based on experimental data of a single clone (or multiple subsets that are based on experimental data of a single clone each). The method comprises training 211 a first component of the at least one machine-learning model using the training data (e.g., the entire training data). The first component represents a generic kinetic behavior of the biological organism. The method comprises training 212 a second component of the at least one machine-learning model using (only) the subset of the training data. Thus, the second component representing a clone-specific kinetic behavior of the biological organism.

Fig. 2c shows a schematic diagram of an example of a computer system 20. The computer system 20 comprises circuitry that is configured to provide the functionality of the computer system 20. For example, the computer system 20 of Fig. 2c comprises (optional) interface circuitry 22, processing circuitry 24, (optional) memory circuitry 26 and storage circuitry 28. For example, the processing circuitry 24 may be coupled with the interface circuitry 22, with the memory circuitry 26 and with the storage circuitry 28. For example, the processing circuitry 24 may be configured to provide the functionality of the computer system 20, in conjunction with the interface circuitry 22 (for exchanging information, e.g., with other components of the computer system or outside the computer system), the memory circuitry 26 (for temporarily storing information, such as machine-readable instructions) and the storage circuitry 28 (for permanently or semi-permanently storing information, such as the machine-readable instructions). In general, the functionality of the processing circuitry 24 may be implemented by the processing circuitry 24 executing machine-readable instructions. Accordingly, any feature ascribed to the processing circuitry 24 may be defined by one or more instructions of a plurality of machine-readable instructions. The computer system 20 may comprise the plurality of machine-readable instructions, e.g., within the memory circuitry 26 or within the storage circuitry 28.

In some examples, the computer system 20 is configured to perform the method of Figs. 1a and/or 1b. Alternatively, or additionally, the computer system 20 may be configured at least one of the methods of Figs. 3, 4, 6 and 9.

In general, machine learning refers to algorithms and statistical models that computer systems can use to perform a specific task without using explicit instructions, instead relying on models and inference. For example, in machine-learning, instead of a rule-based transformation of data, a transformation of data may be used, that is inferred from an analysis of historical and/or training data. For example, the content of images may be analyzed using a machine-learning model or using a machine-learning algorithm. In order for the machine-learning model to analyze the content of an image, the machine-learning model may be trained using training images as input and training content information as output. By training the machine-learning model with a large number of training images and associated training content information, the machine-learning model "learns" to recognize the content of the images, so the content of images that are not included of the training images can be recognized using the machine-learning model. The same principle may be used for other kinds of data as well: By training a machine-learning model using training data and a desired output, the machine-learning model "learns" a transformation between the data and the output, which can be used to provide an output based on non-training data provided to the machine-learning model.

In the present case, the at least one machine-learning model is trained to model kinetic aspects of a biological organism. In this context, the biological organism may be a cell (or rather plurality of cells of the same clone of a cell-line) that is hosted in a bioreactor. However, the proposed concept is not limited to cells. Different types of biological organisms may be supported. The "kinetic aspects" refer to the metabolism of the biological organism, i.e., how a given influx of metabolites and a given environment (extracellular metabolite concentrations, temperature etc.) leads to a certain outflux, which is based on the metabolism of the biological organism. In the context of the GKCM (which may be based on the at least one machine-learning model), instead of influx and outflux, the rates $\mu(t)$ and $r(t)$ are used, with $\mu(t)$ referring to the biomass growth rate, discounted by the cell death, and $r(t)$ being the biomass-specific consumption/production rate. The rates $\mu(t)$, $r(t)$ are possible outputs of the kinetics given by the machine-learning model(s) and the stoichiometry, as shown in the first and second architecture introduced above. In other words, the at least one machine-learning model may model the kinetics of the biological organism, in combination with the flux modes of the biological organism. This is expressed by $f^{kinetic}$, which is the kinetic relation trained in the at least one machine-learning model combined with the stoichiometry from the metabolic network model, with

$$[\mu(t), r(t)]^T$$
$$= f^{kinetic}\left(M^{generic}, M^{clone-specific}, t, x^{envirome}(t), \theta^{generic}, \theta^{clone-specific}\right).$$

In this formula, $M^{generic}$ relates to the generic flux modes, $M^{clone-specific}$ relates to the clone-specific flux modes, and $x^{envirome}(t)$ represents the environment of the biological organism, and thus the input of the at least one machine-learning model. $\theta^{generic}$ and $\theta^{clone-specific}$ are the trainable parameters, and are thus the object of the training of the at least one machine-learning model, and thus of the training of the GKCM and DT.

The training algorithm may be used to adjust (and improve) the (trainable) variables of the Digital Twin (DT), such that it is able to predict the experimental data (e.g., time-resolved concentration of compounds) of all given processes precisely. This learning procedure may particularly include the adjustment and improvement of parameters from the generalized kinetic cell model (i.e., the at least one machine-learning model). Such a trained DT may then be able to predict the kinetics of the cell based on its environment (mainly extracellular metabolite concentrations). The machine-learning model (e.g., the neural network) receives the data of a certain time step and adjusts its weights and biases aiming to predict the experimental data of that specific time step. This may lead to a recurrent approach across all time steps. Thus, in some examples, the machine-learning model may be deemed a recurrent metabolic network. More information on the recurrent metabolic network can be found in WO 2020/224779 A1.

The at least one machine-learning model is trained on the experimental data to learn the cell culture dynamics over time through an optimization procedure. This experimental data is the above-referenced training data, with the at least one machine-learning model being trained on the training input data. A popular approach for training machine-learning models, which may also be used to train the at least one machine-learning model, is called "supervised learning" In supervised learning, the machine-learning model is trained using a plurality of training samples, wherein each sample comprises a plurality of input data values (i.e., training input data), and one or more desired output values (i.e., training output data), i.e., each training sample is associated with a desired output value. By specifying both the training input data and the training output data, the machine-learning model "learns" which output data to provide based on input data that is similar to the samples provided during the training.

In the proposed concept, the training data may comprise such training input data and training output data. In this case, the training input data may comprise a representation of an experimental environment of the organism. The training output data may represent kinetic properties observed in response to the respective experimental environment. As outlined above, this representation of the kinetic properties observed in response to the respective experimental environment may correspond to the rates $\mu(t)$, r(t). However, these rates might not be output directly by the at least one machine-learning model (e.g., by the first and second component). Instead, the first and second component may be embedded in a function (e.g., $f^{kinetic}$), with the first and second component being part of the function (e.g., as kinetic cell model parameters $\theta_{1,3}^{generic}$, $\theta_{2,4}^{clone-specific}$), as well as the generic and clone-specific flux modes $M^{generic}$, $M^{clone-specific}$ and the representation of the experimental environment ($x^{envirome}(t)$). Two examples of possible architectures for implementing such a function are referenced above as the first and second architecture. For example, the first and second component of the at least one machine-learning model may correspond to $f_1$(comprising generic kinetic cell model parameters $\theta_1^{generic}$) and $f_2$(comprising clone-specific kinetic cell model parameters $\theta_2^{clone-specific}$), respectively, with the at least one machine-learning model further comprising a third component $f_3$(comprising generic kinetic cell model parameters $\theta_3^{generic}$) and/or a fourth component $f_4$(comprising clone-specific kinetic cell model parameters $\theta_4^{clone-specific}$). In some cases, the first, second, third and fourth component are assumed to comprise separate machine-learning models (e.g., ANNs). However, these components may also be implemented as separate layers of the same machine-learning model (e.g., ANN). Accordingly, the first and second component (and optional third and fourth component) of the at least one machine-learning model may be separate machine-learning models, or the first and second component (and optional third and fourth component) of the at least one machine-learning model may be a first and a second (and an optional third and fourth) plurality of layers of the same deep neural network. In both cases, the at least one machine-learning model may comprise at least one deep neural network, with the first, second, (optional) third and (optional) fourth component comprising a first, second, third and fourth plurality of layers of the at least one deep neural network.

Supervised learning is generally based on a supervised learning algorithm, e.g., a classification algorithm, a regression algorithm or a similarity learning algorithm. In the present context, a regression algorithm may be used, as regression algorithms may be used when the outputs may have any numerical value (within a range).

Alternatively, reinforcement learning may be used to train the at least one machine-learning model. In reinforcement learning, one or more software actors (called "software agents") are trained to take actions in an environment. Based on the taken actions, a reward is calculated. Reinforcement learning is based on training the one or more software agents to choose the actions such, that the cumulative reward is increased, leading to software agents that become better at the task they are given (as evidenced by increasing rewards).

Both supervised learning and reinforcement learning are based on gradually reducing a deviation between a desired output of the at least one machine-learning model and the output provided by the at least one machine-learning model during the training. In the present context, this output is input into the above-referenced formula, such that the training may be based on gradually reducing a deviation between a desired output of the function (comprising the at least one machine-learning model) and the output provided by the function during the training. Accordingly, training the at least one machine-learning model may comprise determining a deviation between a function and the training output data, with the function being based on the at least one machine-learning model, a first set of flux modes (e.g., $M^{generic}$) representing generic functionality of the plurality of clones of the biological organism and a second set of flux modes (e.g., $M^{clone-specific}$) specific to the single clone of the biological organism (which are also part of the above-referenced function). In other words, training the at least one machine-learning model may comprise determining a deviation between an output of a function, with the function being based on the at least one machine-learning model, the first set of flux modes (e.g., $M^{generic}$) representing generic functionality of the plurality of clones of the biological organism and the second set of flux modes (e.g., $M^{clone-specific}$) specific to the single clone of the biological organism, and the training output data (e.g., the rates observed during the experiment). For example, the first and second set of flux modes may comprise elementary flux modes and/or metabolic base functionalities.

In an example, which is discussed in general terms in WO 2020/224779 A1, the aforementioned function may be based on or correspond to a hybrid model, with the hybrid model comprising the at least one machine-learning model, the first and second set of flux modes and other components, such as a reactor model.

In various examples of the present disclosure, the first and second component are both part of a function, such as $f^{kinetic}$, with the deviation being determined between the training output data and the function. Accordingly, the output of the first component also affects the training of the second component (and vice versa). The same is true for the optional third and fourth components. Accordingly, the first and second (and optional third and fourth) component may be trained based on an output of the respective other component(s).

In the second architecture introduced above, the outputs of the first and second component ($f_1$ and $f_2$, respectively) are input into another component of the at least one machine-learning model, $f_3$ comprising kinetic cell model parameters $\theta_3^{generic}$. This component may be the third component of the at least one machine-learning model, taking an output of the first and second component at its inputs. The method may thus further comprise training 213 the third component of the at least one machine-learning model using the training data (using the same techniques as introduced in connection with the first and second component). Additionally, or alternatively, the at least one machine-learning model, may further comprise the fourth component (e.g., $f_4$ comprising kinetic cell model parameters $\theta_4^{clone-specific}$), which represents one or more flux modes not represented by the first and/or second component. Again, the method may comprise training 214 the fourth machine-learning model using the training data.

The training of the at least one machine-learning model is based on reducing the deviation between the desired output of the at least one machine-learning model (or of the function, in the present case) and the training output data. This deviation may be used as part of a loss function (in case of supervised learning), with the loss being higher the greater the deviation, or as part of a reward function (in case of reinforcement learning), with the reward being higher the smaller the deviation.

In the following, the focus is on supervised learning, with a corresponding loss function. However, similar results may be achieved using reinforcement learning with a corresponding reward function.

In various examples, the at least one machine-learning model may be trained using a stochastic algorithm. For example, the result of the loss function may be reduced or minimized using a stochastic gradient descent algorithm. In each training iteration, a subset of the experimental data / training data (a "batch" in machine learning terminology) is used to compute the parameters of the machine-learning model (e.g., the gradients of the layers of the at least one neural networks). For example, the parameters / gradients may be updated by reducing or minimizing the following loss function:

$$loss = \frac{1}{N} \sum_{p,t,m_i} \left( \frac{c_{p,t,m_i}^{predicted} - c_{p,t,m_i}^{measurement}}{c_{p,t,m_i}^{measurement,std}} \right)^2 + \lambda$$

$$\cdot \sum_{l} |W_l| + \gamma$$

$$\cdot \sum_{p,t,m_i} relu\left(-c_{p,t,m_i}^{predicted} \cdot V_{p,t}\right) + c_{rr}$$

$$\cdot \frac{1}{\sum_{p,m_{exchange,i}} 1} \sum_{p,m_{exchange,i}} \frac{\sum_t \left( r_{p,t,m_{exchange,i}}^{predicted} - r_{p,t-\Delta t,m_{exchange,i}}^{predicted} \right)}{\sum_t r_{p,t,m_{exchange,i}}^{predicted}}$$

where

$c^{predicted}$    are the calculated concentrations by the hybrid model

$c^{measurement}$    are the experimentally measured concentrations

$c^{measurement,std}$    are the standard deviations of the experimentally measured concentrations

$p$    is a cultivation run (i.e., a process)

$t$    is a measurement time point

$m_i$ is metabolite

$m_{exchange,i}$ is exchange metabolite

$\Delta t$ time interval between the two adjacent points on the time grid

$\lambda$ is a weighting factor for regularization of the synaptic weights

$|W_l|$ is the L1 Norm of the synaptic weight between the layer and of the neural network

V is bioreactor volume

$\gamma$ is a weighting factor for regularization of negative amounts

*relu* is the rectifier function

$r^{predicted}_{p,t,m_{exchange,i}}$ is the predicted rate of the exchange metabolite $m_{exchange,i}$ in cultivation run p at time point t

$c_{rr}$ rate-change regularization penalty factor $N = \Sigma_{p,t,m_i} 1$

In the above example, $c^{predicted}_{p,t,m_i} - c^{measurement}_{p,t,m_i}$ may represent the aforementioned deviation.

The first and second (and optional third and fourth) component are trained using the training data, which is based on experimental data of a plurality of clones of the biological organism. This training data is thus not generated based on experimental data of a single clone, but of a plurality of different clones. These clones may all be clones of the same cell line. In other words, the training data may be based on experimental data of a plurality of clones of the same cell-line of the biological organism. Alternatively, clones of two or more different cell-lines (e.g., with one or more clones per cell-line, such as a plurality of clones per cell line) may be used. In other words, the training data may be based on experimental data of a plurality of clones of a plurality of different cell-lines of the biological organism. Moreover, to further increase the breadth of the training data, training data representing different process scales may be used (e.g., experimental data from small-scale experiments, experimental data from medium-scale experiments and data from large-scale production runs). In other words, the training data may be based on experimental data from a plurality of different process scales.

For the purpose of training the second (and optional fourth) component, only a limited subset of the training data is used, i.e., the subset of training data that is based on experimental data of a single clone. Thus, this component or components are trained to represent the clone-specific kinetic behavior of the biological organism, i.e., of the clone being represented by the subset of training data. For example, the second and optional fourth component may be frozen (i.e., set to a non-trainable state) when the at least one machine-learning model is trained with training data outside the subset, i.e., training that does not relate to the single clone.

In the present disclosure, the representation of the experimental environment, $x^{envirome}(t)$), may be the input of the at least one machine-learning model (e.g., of the first, second and fourth component), while $f_1, f_2, f_3$ and $f_4$ may be the respective outputs of the respective first, second, third and fourth components, which are combined in $f^{kinetic}$. In some examples, this representation may be compressed. Thus, the representation of the experimental environment may correspond to a compressed representation of the experimental environment (e.g., $x^{envirome}(t)$) having a reduced dimensionality compared to an uncompressed representation of the experimental environment.

In the previous examples, the rates $\mu(t)$, $r(t)$ have been the main focus of the discussion. However, the DT may be particularly useful when used as part as a bio-pharmaceutical production planning and improvement or optimization. Therefore, the training output data may further represent one or more bio-pharmaceutical product quality properties (e.g., the PQAs introduced above) of the biological organism in an experimental environment.

In some examples of the present disclosure, a so-called Ensemble method may be employed. The Ensemble method is a way to assess the uncertainty in predictive models. In this approach, not a single model, but an ensemble of models may be trained using the same set of training data. To impose variability among different models, a randomly chosen seed value may be set corresponding to each model. This leads to variability in model training/predictions, despite having the exact same training set for all the models within an ensemble. Such a variability in model predictions can then be interpreted as the model uncertainty. Although the ensemble method is computationally expensive due to the training for multiple times, this is a simple approach to quantify the uncertainty of a predictive model.

In general, stochastic features cause variability across trained models when more than one training is performed (with a randomly chosen seed value per each training). This variability across the trained models can be used to quantify training uncertainty. A trained Digital Twin (DT) may include one or more stochastic features such as:

- optimization (stochastic gradient descent)
- random initialization of the DT parameters (e.g., sampled from a uniform distribution)
- dropout (switching off a fraction of neurons randomly)

To quantify uncertainty of a trained DT (and in particular of the at least one machine-learning model of the GKCM), multiple (e.g., 10) generalized kinetic cell models may be trained using the best hyper-parameters found in a greedy hyper-parameter search and with the exact same data set. Here, the entire data set is used (i.e., no train-test or train-validation split is performed, so 100% of the data set is used). Each training is performed considering a randomly chosen seed value. Accordingly, the at least one machine-learning model (e.g., the first, second, optional third and optional fourth component) may form a set of machine-learning models. The method may comprise training 210 a plurality of sets of machine-learning models. The plurality of sets of machine-learning models may be recurrently trained with different seed values, for example. For example, the different seed values may affect at least one of a random initialization of parameters and a dropout of the respective machine-learning models. The stochastic features of an DT leads to variability across these multiple trained kinetic cell models. This leads to variability in DT predictions. The overall DT prediction (i.e., descriptive quality) may be considered as the average of the predictions made by the ten kinetic cell models. The uncertainty of the trained DT may then be defined as the standard deviation of the predictions made by the ten kinetic cell models.

The results of the ensemble method may be a trained DT (which includes the multiple generalized kinetic cell models), a quantification of the descriptive quality of a trained DT, and quantification of the uncertainty of DT predictions.

In various examples of the present disclosure, transfer learning is used. Machine-learning (ML) algorithms iteratively train statistical models to describe the correlation between input and output variables. Typically, this is done by providing the models with a number of input datasets (i.e., the training input data) for which the output is known (i.e., the training output data). Based on the difference between expected and calculated output values the parameters of the ML model may be updated in each iteration until convergence is achieved.

Training machine-learning models is very expensive regarding time and costs due to the demand for a high number of qualitative and variate datasets as well as the computational effort that increases with the amount of data and number of trainable parameters. The latter grows with the number of variables in the models.

Transfer learning with warm-starting reduces these efforts by re-using parts of the model and thus decrease the amount of necessary data and computational costs while providing better generalizability of the ML models. This leads to significant time and cost savings in projects as well as a better predictive quality.

In the context of artificial neural networks (ANNs), this can for example mean that a source model was first trained with a certain task and the resulting weights and biases of that network were then used as an initialization (warm-start) for the training of a target model with a different task using the same network structure. Often only parts of the ANN for the new problem are initialized using the previously trained one or new parts can be added to the preexisting ANN to tackle the new problem. In the ideal case, this can lead to a greatly reduced training time for the ANN used in the second problem as the training starts nearer to an improved or optimal set of parameters throughout the network. This also means that fewer data is needed to receive a good training result in the second training.

Transfer learning can be applied in the generalized kinetic cell model (GKCM). Different use cases can be considered here. The first one is actually inherently part of the concept of the GKCM in the sense, that all clones are sharing information among each other using the generic layers of the ANN. Therefore, even the from scratch training of GKCM will always include an aspect of transfer learning among the datasets leading to less data per clone being needed while maintaining a good generalization capability and high predictive accuracy. This approach of training multiple models at the same time is also known as Multi-task learning and an approach typically used in heterogenous transfer learning problems.

As after training it is possible to differentiate between generic and clone-specific behavior. Since the respective layers are separated it is possible to add additional clones to an existing GKCM using the clone specific layers and a combination of transfer learning and warm-starting. In other words, as further shown in Fig. 2b, the method may further comprise adapting 230, using transfer learning, at least the clone-specific second component of the at least one machine-learning model may be based on training data that is based on experimental data of a further single clone. This use of transfer learning in the GKCM could greatly speed up the training for new clones and would also enable training with a lot less data (compared to a stand-alone "standard" DT for that project) as big parts of the generic layers (and generic behaviors) are already trained on other datasets. In such a scenario, the component of the at least one machine-learning model representing the generic component (i.e., the first component) might either be trained further, or, for the sake of speeding up the training, it may be frozen (i.e., set to be non-trainable).

In the following three examples for transfer learning and/or warm starting are given. In the first setup the complete GKCM (generic and clone-specific layers) may be (re-)trained on the same (or optionally augmented) dataset. The application case include the continuation of stopped trainings (e.g., due to loss of the cloud instance), improvement of the already existing GKCM when new data for the same clones is available or version upgrading of the software.

In the second setup the complete GKCM (generic and clone-specific layers) may be retrained on a new clone-specific dataset. The advantage of this approach is that the training can be run with only a reduced dataset. However, the generic layers might not continuously profit from adding more data into the GKCM when using this approach. In such a scenario, the first component and the second component (and optional third and fourth components) may be trained in a first phase of the training and the second component (and optional fourth component) may be trained in a second phase of the training following the first phase of the training, with the first component being frozen during the second phase of the training.

In the third setup where a GKCM is completely retrained with another new clone the complete set of training data (from the new clone and previously existing process data) may be utilized. It may also be advisable to continue the training of the clone-specific layers for previously existing clones, to compensate for changes in the generic layers. These preexisting clone layers can be warm-started just like the generic layers. The main advantage here is the further improvement of the generalizability of the GKCM.

Once the at least one machine-learning model, and thus the GKCM, is trained, it can be used to generate a corresponding Digital Twin comprising said at least one machine-learning model. Accordingly, as further shown in Fig. 2b, the method further may comprise generating 220 the Digital Twin of the biological organism using the trained at least one machine-learning model. In addition to the at least one machine-learning model, other components may be included in the Digital Twin as well, such as the remaining components (e.g., flux modes) of the GKCM, the extracellular reaction model and the osmolality model.

Machine-learning algorithms are usually based on a machine-learning model. In other words, the term "machine-learning algorithm" may denote a set of instructions that may be used to create, train, or use a machine-learning model. The term "machine-learning model" may denote a data structure and/or set of rules that represents the learned knowledge, e.g., based on the training performed by the machine-learning algorithm. In embodiments, the usage of a machine-learning algorithm may imply the usage of an underlying machine-learning model (or of a plurality of underlying machine-learning models). The usage of a machine-learning model may imply that the machine-learning model and/or the data structure/set of rules that is the machine-learning model is trained by a machine-learning algorithm.

For example, the machine-learning model may be an artificial neural network (ANN). ANNs are systems that are inspired by biological neural networks, such as can be found in a brain. ANNs comprise a plurality of interconnected nodes and a plurality of connections, so-called edges, between the nodes. There are usually three types of nodes, input nodes that receiving input values, hidden nodes that are (only) connected to other nodes, and output nodes that provide output values. Each node may represent an artificial neuron. Each edge may transmit information, from one node to another. The output of a node may be defined as a (non-linear) function of the sum of its inputs. The inputs of a node may be used in the function based on a "weight" of the edge or of the node that provides the input. The weight of nodes and/or of edges may be adjusted in the learning process. In other words, the training of an artificial neural network may comprise adjusting the weights of the nodes and/or edges of the artificial neural network, i.e., to achieve a desired output for a given input. In at least some embodiments, the machine-learning model may be deep neural network, e.g., a neural network comprising one or more layers of hidden nodes (i.e., hidden layers), preferably a plurality of layers of hidden nodes.

Alternatively, the machine-learning model may be a support vector machine. Support vector machines (i.e., support vector networks) are supervised learning models with associated learning algorithms that may be used to analyze data, e.g., in classification or regression analysis. Support vector machines may be trained by providing an input with a plurality of training input values that belong to one of two categories. The support vector machine may be trained to assign a new input value to one of the two categories. Alternatively, the machine-learning model may be a Bayesian network, which is a probabilistic directed acyclic graphical model. A Bayesian network may represent a set of random variables and their conditional dependencies using a directed acyclic graph. Alternatively, the machine-learning model may be based on a genetic algorithm, which is a search algorithm and heuristic technique that mimics the process of natural selection. Alternatively, the machine-learning model may be based on a gaussian process, which is a stochastic process.

The interface circuitry 22 or means for communicating 22 may correspond to one or more inputs and/or outputs for receiving and/or transmitting information, which may be in digital (bit) values according to a specified code, within a module, between modules or between modules of different entities. For example, the interface circuitry 22 or means for communicating 22 may comprise circuitry configured to receive and/or transmit information.

For example, the processing circuitry 24 or means for processing 24 may be implemented using one or more processing units, one or more processing devices, any means for processing, such as a processor, a computer or a programmable hardware component being operable with accordingly adapted software. In other words, the described function of the processing circuitry 24 or means for processing may as well be implemented in software, which is then executed on one or more programmable hardware components. Such hardware components may comprise a general-purpose processor, a Digital Signal Processor (DSP), a micro-controller, etc.

For example, the memory circuitry 26 or memory 26 may comprise volatile or non-volatile memory, e.g., circuitry for providing volatile or non-volatile memory. For example, the memory circuitry or memory 26 may be random-access memory (RAM), such as dynamic random-access memory (DRAM) or static random-access memory (SRAM), or persistent memory (PMEM). For example, at least a portion of the memory circuitry may be part of the processing circuitry, e.g., registers of the processing circuitry.

For example, the storage circuitry 28 or means for storing information 28 may comprise at least one element of the group of a computer readable storage medium, such as a magnetic or optical storage medium, e.g., a hard disk drive, a flash memory, Floppy-Disk, Random Access Memory (RAM), Programmable Read Only Memory (PROM), Erasable Programmable Read Only Memory (EPROM), an Electronically Erasable Programmable Read Only Memory (EEPROM), or a network storage.

For example, the computer system 20 may be a workstation computer system (e.g., a workstation computer system being used for scientific computation).

More details and aspects of the training of the at least one machine-learning model, and thus the GKCM and DT, are mentioned in connection with the proposed concept or one or more examples described above or below (e.g., Fig. 1, 3 to 12). The training of the at least one machine-learning model, and thus the GKCM and DT, may comprise one or more additional optional features corresponding to one or more aspects of the proposed concept, or one or more examples described above or below.

In the following, some examples are given on the application of such a Digital Twin, which may comprise the at least one machine-learning model trained as shown in connection with Figs. 2a to 2b.

In some examples, the Digital Twin may be used for the improvement or optimization of processes.

In general, there are different approaches for process improvement or optimization. Using the trained Digital Twin (DT), e.g., from the Ensemble method, one (e.g., a single) or more processes may now be improved or optimized, e.g., by performing media improvement or optimization and/or feed improvement or optimization. The better the predictive quality of the trained DT, the more reliable are the improvement optimization results. Media improvement or optimization involves improving or optimizing the concentration of compounds in the feed media and/or basal medium with the aim to reduce or minimize a cost function subject to a set of feasibility constraints. Feeding improvement or optimization involves improving or optimizing the feeding strategy (i.e., deciding when to feed and how much to feed) with the aim to reduce or minimize a cost function subject to a set of feasibility constraints.

Additionally, model-based design of experiments (MbDoE) can be performed using the trained DT. In other words, as further shown in Fig. 2b, the method may comprise determining 230 a plurality of experiments to be performed using the biological organism, and continuing training 210, 211, 212, 213, 214 of the at least one machine-learning model based on further training data that is based on the plurality of experiments. The model-based design of experiments can also be called Digital Twin-based design of experiments (DTDoE) and can perform the simultaneous media and feed improvement or optimization as well as the design of new experiments. Thus, the determination 230 of the plurality of experiments may also be part of the method of Fig. 3. In both cases, a set of feasibility constraints may be applied to ensure that a pragmatic solution is obtained.

In the former case (feed/media improvement or optimization), the objective may be to reduce or minimize a cost function (as in the case of media and feed optimization) while in the latter (model-based design of experiments), the experimental data may be enriched in terms of its information contents. Ideally, the experimental data covers a wide range of variable dynamics during a process. Training a DT using such informative experimental data helps to construct a better DT in terms of its descriptive/predictive quality. Having the DT trained, e.g., using the Ensemble method, the trained DT may be used to perform the DTDoE, taking the experimental design constraints into account. The outcome of the DTDoE are instructions for the designed experiments which are proposed for the experimental implementation. The designed experiments may include changes in feeding strategy and/or media compositions. The expert tasked with implementing the experiments may then implement the designed experiments according to the instructions and may provide the resulting experimental data to be used for continuing the training of the at least one machine-learning model. These experimental data may be integrated to the already existing experimental data which were used at the first place to train the DT. Training the DT using such expanded data set may lead to a better DT in terms of their descriptive/predictive quality.

As outlined above, the DT may be used for the purposes of identifying target parameters, such as feed strategy, media composition etc.

Fig. 3 shows a flow chart of an example of a method for determining at least one target parameter of at least one bioreactor comprising at least one biological organism. The method comprises determining 310 the at least one target parameter using at least one Digital Twin of the at least one biological organism that is generated according to the above method (of Fig. 2b) and at least one corresponding cost function. Such a target parameter may, for example, include at least one of a target media composition of a feed medium for the at least one biological organism, a target feeding strategy for the at least one biological organism, a target outflux strategy for the at least one biological organism, and target initial conditions for the at least one biological organism (which may all influence the medium hosting the biological organism). For example, the target parameter may be determined such, that one or more properties of the resulting process (such as product quality, yield of target product etc.) are improved compared to other values of the target parameter. Evidently, the method may also comprise generating 220 the DT as a preparatory task.

In some examples, the at least one target parameter may be determined for a single clone. For this, a single Digital Twin representing the single clone may suffice. Alternatively, the at least one target parameter may be determined for multiple clones of the same cell-lines or of different cell-lines (e.g., for an entire platform being used to host different clones). In this case, the at least one target parameter may be commonly determined for at least two biological organisms using at least two Digital Twins of the at least two biological organisms (i.e., the same parameter or parameters are determined for two or more different biological organisms, i.e., two or more different clones).

Examples of respective cost functions, e.g., for determining target media composition and/or feeding strategy, will be given in the following, along with corresponding constraints for the respective target parameter. For example, the respective cost function can be computed for a given clone or set of clones of the biological organism, for example, and may be used for selection of the optimally performing clone of a given set of clones of a biological organism (e.g., as shown in connection with Fig. 9). For example, the method may comprise computing and scaling the media composition cost function per model of the Digital Twin of the biological organism in order to determine am improved or optimal target parameter (e.g., media composition) across all models of the Digital Twin.

In various examples, some feasibility constraints are applied to facilitate the determination of the at least one target parameter.

For example, constraints regarding the medium (or media) may be applied. The metabolite concentrations in the media (i.e., $c_m$) may be constrained by specific lower and upper bounds, which can be specified as follows:

$$LB_m \leq c_m \leq UB_m$$

The default lower and upper bounds for the basal and feed media composition may be defined using the following rules, for example:

$$LB_m = LBF_m * \left( c_m^{ref} \right)$$

$$UB_m = max \left( c_m^{ref}, min \left( SF_m * S, UBF_m * \left( c_m^{ref} \right) \right) \right)$$

where

m      represents a certain metabolite in a feed medium (i.e., the basal media or the feed media)

$LB_m$      refers to the lower bounds of the basal/feed media concentrations

$UB_m$      refers to the upper bounds of the basal/feed media concentrations

$LBF_m$      refers to the weights/factors used for adjusting the lower bounds of the basal/feed media composition

$UBF_m$      refers to the weights/factors used for adjusting the upper bounds of the basal/feed media composition

$SF_m$      refers to the solubility factor of all the metabolites present in either feed/basal media

$S$      refers to the solubilities of all the metabolites present in either feed/basal media

$c_m^{ref}$      refers to the reference feed/basal media concentration

Note that the lower and upper bound values only represent the default values and can easily be adjusted as per the project specifications by the user.

For example, constraints regarding the feed may be applied. For example, bolus shot volumes may be constrained by specific lower and upper bounds, which can be specified as follows:

$$LB \leq v_{feed} \leq UB$$

Like media composition, the default bounds on the feeding volumes may be defined as follows, for example:

$$LB = LBF * v^{ref}$$

$$UB = UBF * v^{ref}$$

where

$LB$      refers to the lower bounds on the feeding volumes

*UB*     refers to the upper bounds on the feeding volumes
*LBF*    refers to the factors used for defining the lower bounds on the feeding volumes
*UBF*   refers to the factors used for defining the upper bounds on the feeding volumes
$v^{ref}$   refers to the reference feeding volumes

Note that just like for the media constraints, the bounds can be easily adjusted by the user to match the project requirements.

In the following, an example of a loss function for media and feed improvement or optimization is given. For example, the loss function may comprise an objective and a penalty term:

$$Loss = \alpha \cdot c_m(p,t) + \sum_i V_i$$

where

$c_m(p, t)$   is concentration of the metabolite m at time-point $t$ for process $p$.

$c_m(p,t)$   can e.g., represent concentration of the product at the last time point (i.e., $c_m(p,t_{end})$) corresponding to a given process p.

$\alpha$   is the mapping function for the objective term. It could take any one of the following values:

$$\alpha = \begin{cases} +1 \text{ minimize } c_m(p,t) \\ -1 \text{ maximize } c_m(p,t) \end{cases}$$

$V_i$   is the penalty term *i*.

The penalty term depicts the amount of violation of a process specification (such as concentrations, volumes, osmolality etc.) from a desired range or a target value. Mathematically, this can be stated as follows:

$$V_i = w_i \cdot D_i$$

where

$W_i$   refers to penalty imposed on the deviation of process specification *i*
$D_i$   refers to the amount of deviation.

The different violation terms may include one or more of the following:

- negative concentration violation: This term may ensure that the metabolite concentrations remain positive and are therefore realistic.
- upper and lower vessel volume violations: This term may ensure that the bioreactor volume stays within or in close proximity to the specified range as defined by the upper/lower limits.
- upper and lower concentration violations: This term may ensure that the concentration of compounds in the bioreactor stays within or in close proximity to the specified range as defined by the upper/lower limits.
- media deviation violation: This term may ensure that the concentration of compounds in the feed media do not unnecessarily deviate from the reference values, thereby, making sure that only the concentration of metabolites that have a significant impact on the loss function vary during the optimization. This may help in eliminating redundant changes in the feed media optimization.
- $c_0$ deviation violation: This term may ensure that the concentration of compounds in the basal media do not deviate unnecessarily from the reference values, thereby supporting that only the concentration of metabolites that have a significant impact on the loss function vary during the optimization. This may help in eliminating redundant changes in the basal media optimization.
- *Im* output violation: This term may ensure that the osmolality of the optimized media remains below a pre-defined value. High osmolality values may put risk on the cell health.
- feed deviation violation: This term may ensure that the feed volumes do not deviate unnecessarily from the reference values, thereby supporting that only those regions of the feeding profile that have a significant impact on the loss function vary during the optimization. This may help in eliminating redundant changes in the feed optimization.

- total feed volume violation: This term may ensure that the total feed volume stays within or in close proximity to the specified range as defined by the upper/lower limits.

In the following, an example of a loss function for experimental design (i.e., MbDoE/DTDoE) is given. The loss function may be defined as:

$$Loss = \sum_m \sum_n \alpha \cdot \rho_{m,n}^2 + \sum_i V_i$$

where $\rho_{m,n}$ is the correlations factor between the predicted concentrations of metabolites *m* and *n* which is mathematically defined as:

$$\rho_{m,n} = \frac{\sigma_{mn}}{\sigma_m \cdot \sigma_n} = \frac{\sum_{p,t}(c_m(p,t) - c_m^-) \cdot (c_n(p,t) - c_n^-)}{\sqrt{\sum_{p,t}(c_m(p,t) - c_m^-)^2} \cdot \sqrt{\sum_{p,t}(c_n(p,t) - c_n^-)^2}}$$

where

$c_m(p, t)$ and $c_n(p, t)$ are the predicted concentrations of metabolites *m* and *n* $\overline{c_m}$ and $\overline{c_n}$ are the average metabolite concentrations for metabolites *m* and *n*, computed across the processes and timepoints.

with the definitions of $\alpha$ and $V_i$ as in the previous section.

The improvement or optimization problems may be solved using a computational optimizer, such as the ADAM optimizer (Kingma, D.P. and Ba, J., 2014. Adam: A method for stochastic optimization. arXiv preprint arXiv: 1412.6980), which may be employed to solve the above stated improvement or optimization problems. The solving of the problem may involve one or more of the following three components: 1) an ensemble optimization, 2) a multi-start, and 3) a piecewise constant improvement or optimization, each of which are discussed below.

In the following, more details are given on the ensemble optimization or improvement technique. As stated earlier, a trained DT may be employed as part of the improvement or optimization framework. When the Ensemble method is employed, a trained DT includes several kinetic cell models. In a general setting, one may solve an optimization problem for each of these kinetic cell models, thereby obtaining a plurality of different improved or optimal solutions. The next task would then be to combine these solutions in order to obtain one single overall improved or optimal solution. However, this is not at all trivial and may lead to significant loss of information, thereby providing a sub-optimal solution.

To avoid this issue, an ensemble-based optimization approach may be employed. This also involves the use of the plurality of kinetic cell models in a trained DT, obtained from the Ensemble method. However, instead of solving one optimization problem for each of these in a sequential manner, all kinetic cell models may be incorporated as part of the single improvement or optimization framework. This may be done by defining an overall objective of the proposed improvement or optimization framework, which may be a function of the plurality of objective function values resulting from the plurality of kinetic cell models. Thus, the problem may be now to find an improved or optimal solution that reduces or minimizes the combined cost function. Mathematically, this can be stated as follows:

$$Overall\ Loss = f(Loss_1, Loss_2, \ldots, Loss_N)$$

where $Loss_i$ is the loss function computed using the kinetic cell model model *i*. Typically, the function *f* is the average function and hence, the overall loss for *N* kinetic cell models can be stated as follows:

$$Overall\ Loss = \frac{\sum_i Loss_i}{N}$$

Moreover, the aforementioned multi-start approach may be used. The multi-start approach may form a second component of the improvement or optimization framework. As indicated by the name, this means that multiple initial start points for the improvement or optimization problem are used. For example, these initial points may be generated by the help of a quasi-random Sobol sequence. Assuming that these are K points, this leads to K improvement or optimization problems each starting from a different initial value for the concentration of the media components. The multi-start approach may improve exploration of the design space, and hence, avoid getting trapped in a locally optimal region. Thus, the higher the number of initial points, the better is the coverage of the design space, and hence, enhancing the probability of finding a globally improved or optimal solution.

In some examples, a piecewise constant improvement or optimization may be used. Unlike the multi-start approach, the piecewise constant improvement or optimization is only applicable for improvement or optimization of time-resolved components such as feeding strategy. Hence, it might only be applicable for the feed optimization and experimental design. This strategy involves the discretization of the design space into one/many sections. It is then assumed that the design points lying in those sections remain constant i.e., fixed at a particular value. This helps to reduce the complexity of the improvement or optimization problem as it may lead to a significant reduction in the number of decision variables or the size of the design space. Furthermore, it may help obtain a more pragmatic solution with lesser or rather controlled number of variations across the feeding profile.

Considering a single feed with 10 bolus shot volumes, improvement or optimization of the feeding strategy may yield improved or optimal values for these bolus shot volumes. Using the piecewise constant strategy, the algorithm may discretize the ten timepoints into multiple groups and then a constant feeding volume may be assumed in each of these groups. Thus, this may reduce the number of decision variables from number of bolus shots to number of groups. The complexity of the problem may vary depending on the number of groups for a particular feed.

In some examples, glucose control may be performed. Typically, the glucose feed might not be improved or optimized, as most cell culture processes are operated with a threshold-based glucose control. Glucose concentrations in the bioreactor may be influential on the performance of a bioprocess. Therefore, an accurate description of the control mechanism that is applied in the actual process may be important for the accuracy of the predicted optimized bioprocess.

The glucose control feed is generally applied from a medium composed of concentrated glucose. In order to prevent glucose limitations in a given bioprocess, glucose may be fed as soon as the glucose concentration in the bioreactor falls below a given threshold. The corresponding volume addition of the glucose feed depends on the glucose concentration in the feed media and the desired bioreactor concentration of glucose (setpoint). Glucose control is typically applied for a certain control period during the process (i.e., between the "control starts" and "control ends") and with a certain control frequency (e.g., daily control).

Glucose may exist in other feed media too (i.e., other media besides glucose medium). Hence, the glucose concentration in the bioreactor increases when feeding from these other media may be applied. When both, other feeds and the glucose control feed are applied, the other feeds are taken into account as a possible basis for the computation of the required glucose feed is volume addition. As a result, the glucose control feed may close the remaining gap to the setpoint.

This way, the glucose concentration may be kept between the threshold and the setpoint values for a certain time period during the process. Of course, each control period during a running process may take its own threshold and setpoint values. It is also possible to extend the control period to the entire process duration.

Some aspects of the present disclosure may relate to model predictive monitoring and control. Even well controlled manufacturing processes may encounter unknown variables and deviate from acceptable ranges. These deviations are often detected only after they occur, thus providing little opportunity to implement corrective and/or preventive actions (CAPAs). Additionally, measurements might only be available on different timescales (on-, off-, at-, inline), making it difficult to track the process in real-time. Model predictive monitoring and control may enable dealing with different timescales of measurements, estimation of state of the bioprocess, prediction of the performance of a bioprocess based on real-time measurements (monitoring), and improvement or optimization of the process within a defined reference profile ("golden batch") to provide a control advice.

Fig. 4 shows a flow chart of an example of a method for monitoring a biological manufacturing process involving a biological organism. The method comprises determining 410 an estimated state of the biological manufacturing process using a Digital Twin of the biological organism that is generated according to the method of Fig. 1b. The Digital Twin is supplied with information on the environment of the biological organism (i.e., the representation of the environment) using a moving horizon-approach. The method comprises comparing 420 the estimated state of the biological manufacturing process with an observed state of the biological manufacturing process. For example, a moving-horizon estimation algorithm may be used in the comparison of the estimated state of the biological manufacturing process with the observed state of the biological manufacturing process. It is evident, that the method of Fig. 4 may further comprise generating 220 the Digital Twin.

For state estimation, moving horizon estimation (MHE) may be applied where a trained DT is used to obtain predictions which are reconciled based on the difference between predicted and measured metabolite concentrations and the trust that is put on model or measurements. This allows to run prediction timepoint-wise from measurement to measurement by taking into account model uncertainty in terms of model-plant mismatch and measurement noise as well as the estimation of unmeasured metabolite concentrations.

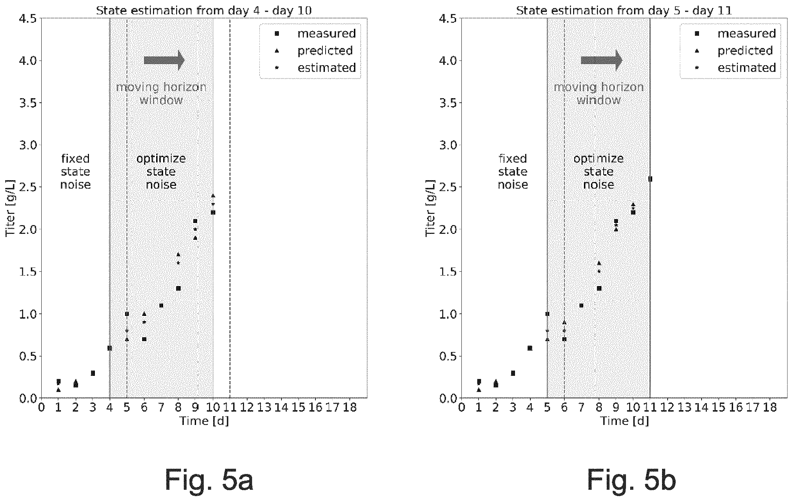

MHE is a finite receding horizon improvement or optimization technique for multivariable estimation that uses a dynamic reactor model, a past trajectory of measurements and a cost function which is to be minimized. The improvement or optimization may be performed for a certain time interval (i.e., the horizon) which is shifted forward in time (i.e., the moving horizon) when new measurements become available (e.g., for a process that takes 14 days with MHE horizon of 6 days (d) and a time grid of 24 hours one optimization could be performed from 0-1d, 0-2d, ..., 0-6d, 1d-7d, 2d-8d, ..., 8d-14d) (see Fig. 5).

Figs. 5a and 5b show schematic diagrams of a moving horizon estimation (MHE) algorithm according to an example for a horizon of 6 days. The algorithm performs a reconciliation (estimated state, star-shaped dots) taking into account measurements (rectangular-shaped dots) as well as predictions (triangular-shaped dots) from the Digital Twin. The state noise for timepoints outside of the moving horizon window may be kept fixed while those inside may be changed during optimization. MHE also allows for the estimation of unmeasured states, see e.g., day 3 or 7. After improvement or optimization and new incoming measurements, the window moves forward, and the procedure may be repeated.

For example, the mathematical problem may be defined as follows:

$$\min_{w,u,c_0} \quad L_{MHE}(\{c\},\{w\})$$
$$\text{s.t.} \quad c_{k+1} = f(c_k, u_k, p_k) + w_k$$
$$c_k \in C, u_k \in U, p_k \in P, w_k \in W$$

where

| | |
|---|---|
| $w$ | is the state noise (describing the model-plant mismatch), |
| $L_{MHE}$ | the cost function, |
| $c$ | the vector of metabolite concentrations including biomass, |
| $C$ | the set of feasible concentrations due to constraints, |
| $k$ | the time(point), |
| $u$ | the input for the Digital Twin except for the reactor concentrations i.e., feeding and sampling, |
| $U$ | the set of feasible inputs due to constraints, |
| $f(\cdot)$ | the functional relation between concentrations, inputs and additional parameters and concentrations at the next timepoint (the Digital Twin model), |
| $p$ | the vector of additional parameters (pH, T, osmolality...), |
| $P$ | the set of feasible parameters due to constraints, |
| $c_k, p_k, u_k$ | the concentrations, parameters, and input at timepoint $k$. It is assumed that there is a model-plant mismatch, in the following referred to as state noise, as well as noisy measurements and inputs (feeding, sampling). |

For example, the cost function may be defined as (adapted from Morabito, Bruno, et al. "Multi-mode Model Predictive Control and Estimation for Uncertain Biotechnological Processes." IFAC-PapersOnLine 52.1 (2019): 709-714)

$$L_{MHE}(\{c\},\{w\}) = \frac{1}{M} \sum_{i=-N_{MHE}}^{t} \left( \sum_{m=1}^{M} \left( \|w_i^n\|_{Q_{MHE}}^2 + \|c_i^m - c_i^{meas}\|_{R_{MHE}}^2 \right) \right)$$

where

| | |
|---|---|
| $m \in M$ | is the number of predictive models of the DT |
| $w_i^m$ | the vector of state noise of model $m$ at timepoint $i$, |
| $c_i^{meas}$ | is the vector of measured concentrations (can also just be a subset of metabolites necessary for the DT) at timepoint $i$, |
| $c_i^m$ | the vector of concentrations predicted by model $m$ at timepoint $i$, |
| $N_{MHE}$ | is the horizon length for state estimation in hours, |

$Q_{MHE}$    is the weighting matrix of the state noise

$R_{MHE}$    is the weighting matrix of the stage cost (measurement noise) and the matrix vector norm $\|x\|_A^2 = x^T \cdot A \cdot x$
.

In some examples, model predictive control (MPC) may be performed. Given a reference trajectory of metabolite concentrations, improvement or optimization during the cultivation may be performed to obtain improved or optimized inputs (e.g., feeding, sampling) that may keep the process close to the reference while keeping the control effort reduced or minimal even if there are deviations in the process run. This may reduce risks during manufacturing while maintaining product quality.

Fig. 6 shows a flow chart of an example of a method for controlling a biological manufacturing process involving a biological organism. The method comprises continuously adapting 610 an environment of the biological manufacturing process using a Digital Twin of the biological organism that is generated according to the method of Fig. 1b, with the Digital Twin being supplied with information on the environment of the biological organism (i.e., the representation of the environment) using a receding horizon approach. The method comprises comparing 620 an estimated state of the biological manufacturing process with a defined reference state trajectory of the biological manufacturing process. For example, a model predictive control algorithm is used in the comparison of the predicted state of the biological manufacturing process with the defined reference state trajectory of the biological manufacturing process. Based on the comparison, the environment may be continuously adapted 610. For example, the state estimation may be performed according to the method of Fig. 4. The improvement or optimization of the environment (i.e., of the target parameters of the environment) may be performed according to the method of Fig. 3. For example, the method of Fig. 6 may further comprise generating 220 the Digital Twin.

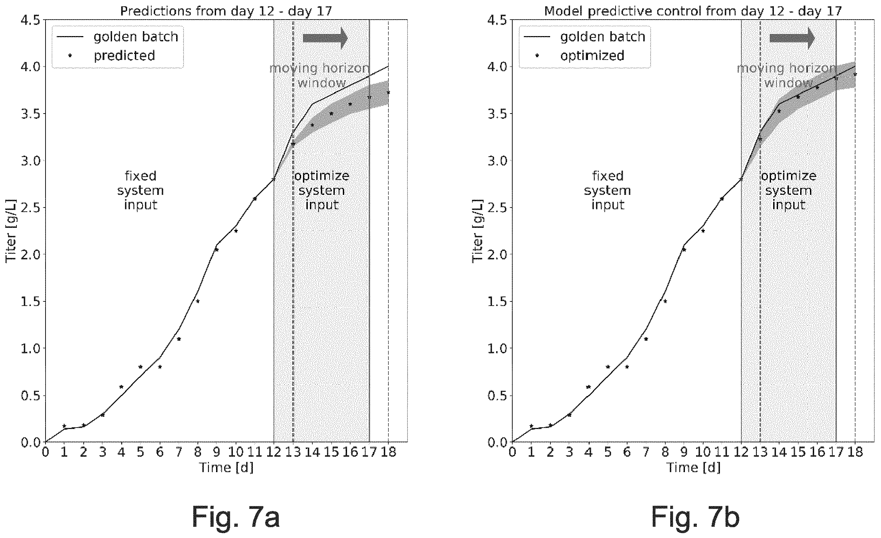

Figs. 7a and 7b show schematic diagrams illustrating the model predictive control (MPC) algorithm according to an example, for a horizon of 5 days. In Figs. 7a and 7b, the algorithm is used to improve or optimize the control input (e.g., feeding volumes or rates) by reducing or minimizing the deviation between reference (solid line) and prediction (star-shaped dots) by the Digital Twin such that the improved or optimized prediction trajectory (the dark funnel around the prediction points) follows the reference (the solid line). Additionally, the control effort as well as the deviation of the terminal cost (here: final titer) may be improved or optimized (not shown in Figs. 7a and 7b). The improvement or optimization may be performed for the predictions into the future within a defined horizon which moves forward during cultivation time. Improved or optimized control input outside of this horizon window may be kept fixed (either implemented or subject to future optimization).

MPC is a finite receding horizon optimization technique that uses a dynamic reactor model, a reference trajectory and a cost function which is to be reduced or minimized with respect to control inputs. The improvement or optimization may be performed for the whole scheduled cultivation time but at the plant only the control for the next timepoint is applied. Then improvement or optimization is performed again etc.

For example, the mathematical problem may be defined as follows:

$$\min_{u} \quad L_{MPC}(\{c\},\{u\})$$
$$\text{s.t.} \quad c_{k+1} = f(c_k, u_k, p_k)$$
$$c_k \in C, u_k \in U, p_k \in P$$

where

$L_{MPC}$    is the cost function,
$c$        the vector of metabolite concentrations including biomass,
$C$        the set of feasible concentrations due to constraints,
$k$        the time(point),
$u$        the input for the Digital Twin except for the reactor concentrations i.e., feeding and sampling,
$U$        the set of feasible inputs due to constraints,
$f(\cdot)$    the functional relation between concentrations, inputs and additional parameters and concentrations at the next timepoint (the Digital Twin model),
$p$        the vector of additional parameters (pH, T, osmolality...),
$P$        the set of feasible parameters due to constraints,
$c_k, p_k, u_k$    the concentrations, parameters, and input at timepoint $k$.

For example, the cost function may be defined as (adapted from Morabito, Bruno, et al. "Multi-mode Model Predictive Control and Estimation for Uncertain Biotechnological Processes." IFAC-PapersOnLine 52.1 (2019): 709-714.)

$$L_{MPC}(\{c\{u\}\}) = \frac{1}{M} \sum_{m=1}^{M} \left( \sum_{i=0}^{N_{MPC}-1} \left( \left\| c_i^m - c_i^{ref} \right\|_{Q_{MPC}}^2 + \|u_i\|_{R_{MPC}}^2 \right. \right.$$

$$\left. \left. + \left\| c_{N_{MPC}}^m - c_{N_{MPC}}^{ref} \right\|_{E_{MPC}}^2 \right) \right)$$

where

$m \in M$     is the set of predictive models of the DT

$i \in N_{MPC}$     is the set of timepoints till the finite horizon $N_{MPC}$ (end of cultivation)

$c_i^m$     is the vector of metabolite concentrations predicted by the DT $m$ at timepoint $i$

$c_i^{ref}$     is the vector of reference metabolite concentrations at timepoint $i$

$u_i$     is the input of the DT, here: feeding (sampling will be kept as in reference process)

$c_{N_{MPC}}^m$     is the vector of metabolite concentrations predicted by the DT $m$ at timepoint $N_{MPC}$ (here end titer), also

$c_{N_{MPC}}^{ref}$     is the vector of reference metabolite concentrations at timepoint $N_{MPC}$ (here end titer)

$Q_{MPC}$     is the weighting matrix (covariance) of the reference deviation

$R_{MPC}$     is the weighting matrix (covariance) of the control effort

$E_{MPC}$     is the weighting matrix (covariance) of the terminal cost and the matrix vector norm $\|x\|_A^2 = x^T \cdot A \cdot x$.

In the following, an overall improvement or optimization problem is discussed.

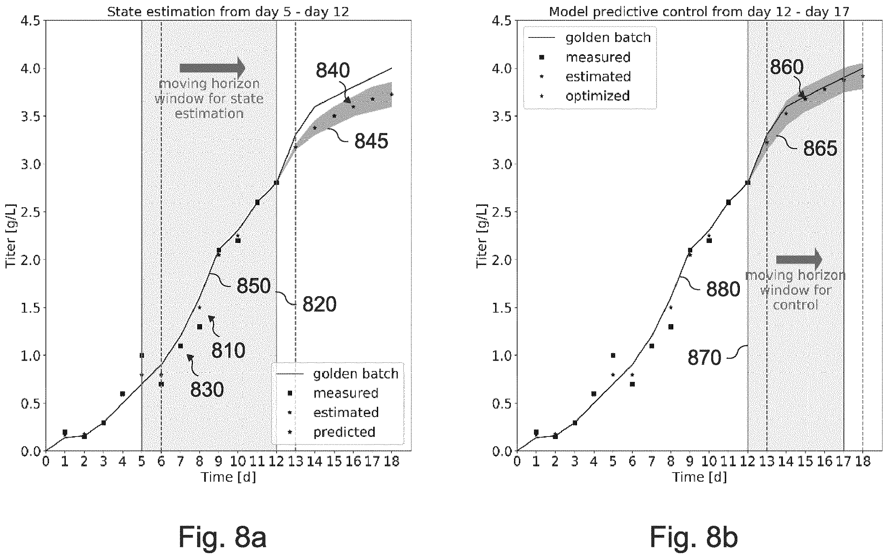

Figs. 8a and 8b show schematic diagrams of a model predictive monitoring and control algorithm for a state estimation horizon of 7 days and a control horizon of 5 days. The algorithm performs a reconciliation ("state noise", stars 810 left of the second horizon line 820, Fig. 8a) taking into account measurements (rectangles 830, Fig. 8a) as well as predictions (stars 840 and area 845 right of the second horizon line, Fig. 8a) from the Digital Twin. The state noise for timepoints outside of the moving horizon window are kept fixed while those inside are changed during improvement or optimization. MHE also allows for the estimation of unmeasured states. The state estimate at the latest timepoint may then be used as initial condition for the MPC. The MPC algorithm may improve or optimize the control input (e.g., feeding volumes or rates) by reducing or minimizing the deviation between the golden batch (solid line 850, Fig. 8a) and prediction by the Digital Twin (stars 840 and area 845 right of the second horizon line 820, Fig. 8a) such that the improved or optimized prediction trajectory (stars 860 and area 865 right of the first horizon line 870, Fig. 8b) follows the golden batch (solid line 880, Fig. 8b). Additionally, the control effort as well as the deviation of the terminal cost (here: final titer) may be improved or optimized (not shown in Figs. 8a and 8b). The improvement or optimization may be performed for the predictions into the future within a defined horizon which moves forward during cultivation time. Improved or optimized control input outside of this horizon window may be kept fixed (either implemented or subject to future optimization).

The model predictive monitoring and control algorithm may combine both state estimation via moving horizon estimation as well as model predictive control. At each new incoming measurement, the state estimator may run the MHE algorithm to obtain the latest state estimates within the MHE horizon (e.g., the method of Fig. 4). The current estimated state may then be used as initial condition for the predictions of the MPC controller that improves or optimizes the control input in terms of the MPC objective within the MPC horizon (see Figs. 6, 8a to 8b).

For example, the mathematical problem may be defined as follows:

$$\min_{u} \quad L_{MPC}(\{c\}, \{u\})$$
$$\text{s.t.} \quad c_{l+1} = f(c_l, u_l, p_l)$$
$$c_l \in C, u_l \in U, p_l \in P, l \in [t; t + N_{MPC}]$$
$$c_t = \tilde{c}_t$$

$$\min_{w,u,c_0} \quad L_{MHE}(\{\tilde{c}\}, \{w\})$$
$$\text{s.t.} \quad \tilde{c}_{k+1} = f(\tilde{c}_k, u_k, p_k) + w_k$$
$$\tilde{c}_k \in C, u_k \in U, p_k \in P,$$
$$w_k \in W, k \in [t - N_{MHE}; t]$$
$$\tilde{c}_0 = \tilde{c}(t = 0h)$$

where

$\tilde{c}$    is the vector of estimated metabolite concentrations including biomass

As outlined in connection with Fig. 3, one task during the improvement or optimization of the process may be the selection of a promising clone. Fig. 9 shows a flow chart of an example of a method for selecting a clone of a biological organism. For the purpose clone selection, the method comprises generating 220 a plurality of Digital Twins of a plurality of clones of the biological organism using the method of Fig. 1b. The method comprises selecting 910 the clone by comparing one or more properties, such as product quality, yield of target product (or more general clone potential or clone plasticity, which may comprise the yield of the target product and/or a platform fit of the clone) etc., of the plurality of Digital Twins. In general, clone potential or clone plasticity is a property of a clone which can be different across clones and refers to the potential change in performance (e.g., titer/product yield) of a clone after process conditions are changed. It can be one of the properties used as criterion in clone selection. For example, the potential for improved titer may be evaluated via process improvement or optimization (feeding strategy and or feed media composition) for each clone. Accordingly, the one or more properties may be selected based on target parameters that were determined according to the method of Fig. 3. Accordingly, the method of Fig. 9 may further comprise determining 310 the at least one target parameter for each clone using the plurality of Digital Twins and the corresponding cost function, with the clone selection 910 being based on the determined target parameters.

In the following, a practical example of the proposed concept is given. In this example, it is demonstrated, that the Digital Twin can be trained on data across clones and process scales and be used for model predictive monitoring and control during manufacturing.

First, the experimental data being used for the Digital Twin generation is discussed. An industrial recombinant Chinese Hamster Ovary (CHO) cell line expressing an IgG monoclonal antibody (mAb) through a Glutamine Synthetase (GS) expression system is used in this example. The amino acid composition (in mol-%) of the monoclonal antibody was: Ala 5.0, Arg 2.9, Asn 4.0, Asp 4.0, Cys 2.4, Glu 4.8, Gln 4.7, Gly 6.5, His 2.0, Ile 2.3, Leu 7.4, Lys 6.8, Met 0.8, Phe 3.5, Pro 7.0, Ser 12.7, Thr 8.0, Trp 1.7, Tyr 4.5, Val 9.0.

The experimental data for the Digital Twin generation comprised datasets from a 15ml and 250 ml scale (and thus two different process scales), with a total of 263 cultivations and 31 clones which were cultivated in fed-batch mode producing IgG antibodies including complete amino acid analysis, organic acids, cell count, product titer, NH4 and glucose concentration. For most of the clones, 4 cultivations were available, however in some cases up to 46 cultivations were provided.

A genome-scale CHO-K1 metabolic network model based on Hefzi et al. (Hefzi, Hooman, et al. "A consensus genome-scale reconstruction of Chinese hamster ovary cell metabolism." Cell systems 3, 5 (2016): 434-443) was used. The database was further curated to yield 3,700 reactions and transport steps. Important metabolic functions were validated and further pathways such as glycosylation, mAb production, growth and cell lysis added.

The following metabolic base functionalities were derived: synthesis of non-essential amino acids from glucose and NH4, degradation of amino acids to CO2, synthesis of cysteine from methionine, proline synthesis from arginine, degradation of glucose to CO2, synthesis of pyruvate and lactate from glucose, degradation of pyruvate and lactate to CO2, degradation of serine to pyruvate, synthesis of biomass and mAb, synthesis of tyrosine from phenylalanine and cell death and lysis.

For the extracellular network, the abiotic degradation of glutamine to 5-oxoproline (and NH4, H) was considered.

In the following, the concrete architecture in the GKCM that was used in this example to describe the growth and exchange rates of measured metabolites is discussed. In the following, the term $ANN_j$ is used to describe feed forward neural networks, as function of the specified inputs.

$$[\mu(t), r(t)]^T = M^{generic}$$

$$\cdot f_3\Big(f_1\big(x^{envirome}(t), \theta^{compression}, \theta_1^{generic}\big),$$

$$f_2\big(x^{envirome}(t), \theta^{compression}, \theta_2^{clone-specific}\big), \theta_3^{generic}\Big) + M^{clone-specific}$$

$$\cdot f_4\big(x^{envirome}(t), \theta^{compression}, \theta_4^{clone-specific}\big)$$

where $f_3$ is

$$f_3\Big(f_1, f_2, \theta_3^{generic}\Big)$$

$$= f_1\big(x^{envirome}(t), \theta^{compression}, \theta_1^{generic}\big)$$

$$\cdot \Big(1 + f_2\big(x^{envirome}(t), \theta^{compression}, \theta_2^{clone-specific}\big)\Big) \cdot H_3,$$

$$\theta_3^{generic} = H_3 \geq 0,$$

where $f_1$ is

$$f_1\big(x^{envirome}(t), \theta^{compression}, \theta_1^{generic}\big) = ANN_1\big(\hat{x}^{envirome}(t), \theta_{ANN1}^{generic}\big) \cdot H_1,$$

$$ANN_1 \geq 0, \; H_1 \geq 0, \theta_1^{generic} = \{\theta_{ANN1}^{generic}, H_1\}$$

and $f_2$ is

$$f_2\big(x^{envirome}(t), \theta^{compression}, \theta_2^{clone-specific}\big)$$

$$= max\big(-1, ANN_2\big(\hat{x}^{envirome}(t), \theta_{ANN2}^{clone-specific}\big) \cdot H_2\big),$$

$$H_2 \geq 0, \theta_2^{clone-specific} = \{\theta_{ANN2}^{clone-specific}, H_2\}$$

$ANN_1$ (e.g., the first component of the at least one machine-learning model) was a feed forward neural network with 2 hidden layers and 100 neurons each and an output dimension of 100. A dropout rate of 20% was used on the hidden layers and a parametric relu was used as activation function in the hidden layers. The activation function of the output layer was the sigmoid function. $H_1$ was a positive scalar.

$ANN_2$ (e.g., the second component of the at least one machine-learning model) was a feed forward neural network with 2 hidden layers and 40 neurons each and an output dimension of 100. A dropout rate of 20% was used on the hidden layers and a parametric relu was used as activation function in the hidden layers. The activation function of the output layer was the tanh function. $H_2$ was a positive scalar.

The matrix $H_3$ was a 100 × 1253 matrix with $h_{i,j} \in R_0^+$.

The GKCM in this example only used generic modes $M^{generic}$, no clone-specific modes $M^{clone-specific}$. Hence, $f_4$ is zero in this example. In other words, the fourth component was not necessary.

Furthermore, no explicit representation of the input vector in a lower dimensional space was used, i.e., $\hat{x}^{envirome} = x^{envirome}$.

In the following, the training and validation of the Digital Twin is discussed. Data augmentation was used such that each process was duplicated leading to the original set of processes which was linked to specific clones and another set of processes which was not linked to specific clones. For processes not linked to a specific clone, the output of $f_2$ was 0. As a consequence, a generic behavior could be identified as output of $f_1$ and $f_3$ in combination with $f_2 = f_4 = 0$.

The predictive power of the Digital Twin was evaluated using the 4-fold cross validation set (see Figure 5). Each validation and train set always contained at least 1 cultivation of each clone. I.e., if 4 cultivations were available for a clone, 3 cultivations would be in each train set and 1 cultivation would always be in the validation set.

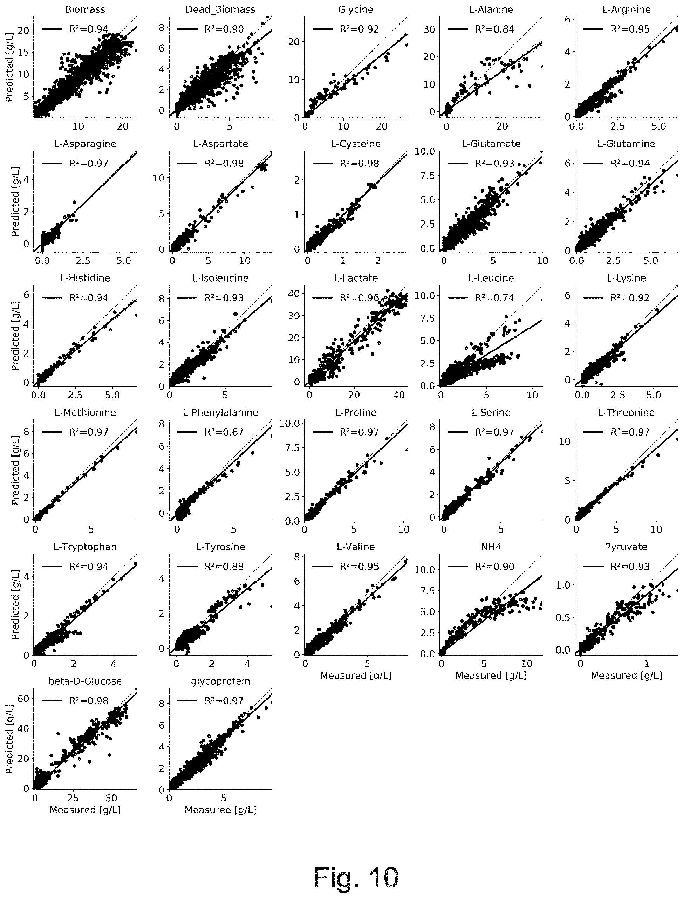

Fig. 10 shows regression plots showcasing a predictive quality of the GKCM per metabolite. The plot compares the measured validation data with the predictions of the GKCM. A $R^2$ of 1.0 indicates a perfect prediction.

Model predictive monitoring and control was implemented on the basis of the Cedex Bio HT (Roche) data to keep the reference cultivation on the improved or optimal target trajectory regarding biomass and IgG concentration via improving or optimizing the feeding profile. State estimation was based on the whole available Cedex Bio HT (Roche) dataset taking into account uncertain initial concentrations, as well as uncertainties in feeding and sampling information by allowing to change these variables during optimization.

In this specific example the aim for the model predictive control was to keep the cultivation on the reference trajectory relying on the predictions and state estimations from the Digital Twin. The state estimates were significantly closer to the real measurements compared to DT predictions alone with the following improvements regarding $R^2$ values for the following metabolites: biomass from 0.63 to 0.9, glutamate from 0.9 to 0.96, glutamine from 0.67 to 0.87, pyruvate from 0.8 to 0.84, glucose from 0.5 to 0.83, glycoprotein (IgG) from 0.85 to 0.98. Biomass and glycoprotein concentrations could be kept closer to the reference trajectory (biomass $R^2=0.79$, glycoprotein $R^2=0.98$) than without control (biomass $R^2=-5.09$, glycoprotein $R^2=0.93$).

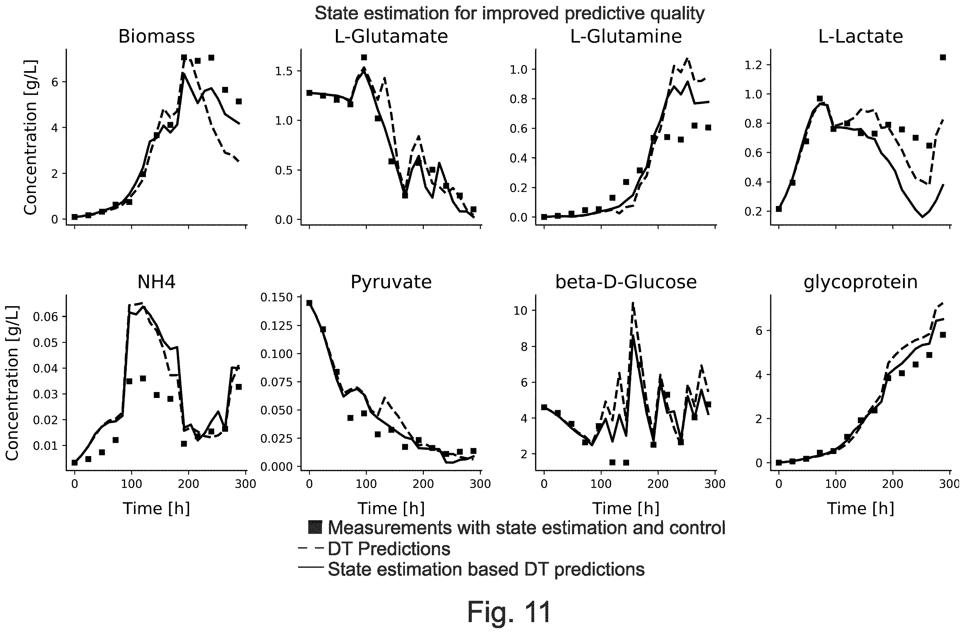

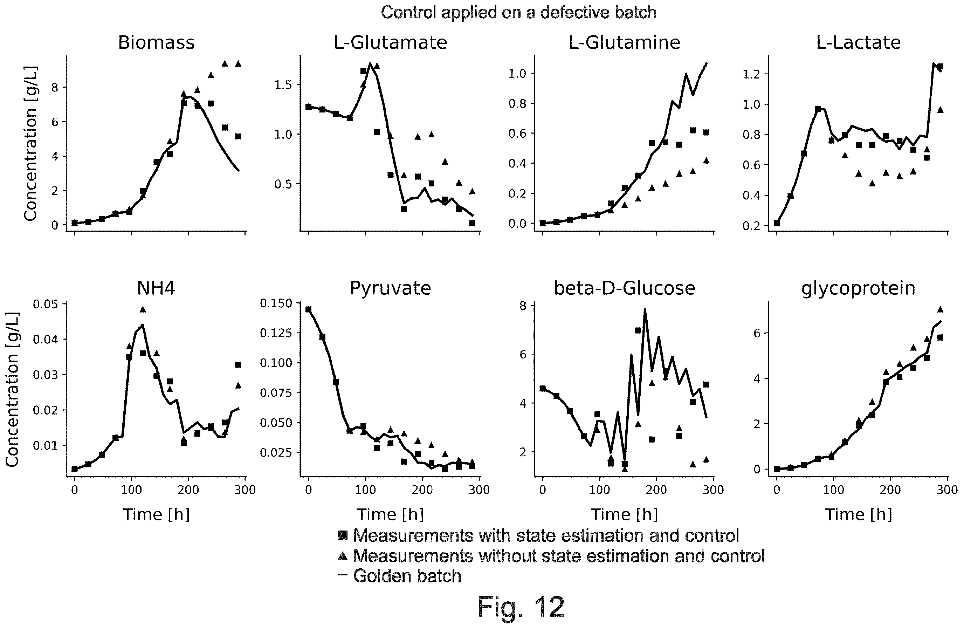

This example illustrates that the Digital Twin, proposed by the proposed concept, can have a high predictive quality on the validation set and provide reliable estimates of the current state (Fig. 11), while keeping the process on the target trajectory (Figure 12), which leads to robust manufacturing.

Fig. 11 shows a schematic comparison of GKCM predictions with and without state estimation as well as corresponding measurement results from a process using state estimation and control. The GKCM predictions using the state estimation are much closer to the actual measurements than the predictions without state estimation, showcasing the improvements achieved through the state estimation.

Fig. 12 shows a schematic diagram showcasing a process which deviated from a golden batch (comparing the measurements of the process with and without control). Using control and state estimation, the process was recovered and resulted in a trajectory close to the golden batch. The objective of the controller were the biomass and glycoprotein trajectories. However, it can be seen that the trajectories of the other metabolites could be improved as well.

The aspects and features described in relation to a particular one of the previous examples may also be combined with one or more of the further examples to replace an identical or similar feature of that further example or to additionally introduce the features into the further example.

Examples may further be or relate to a (computer) program including a program code to execute one or more of the above methods when the program is executed on a computer, processor, or other programmable hardware component. Thus, steps, operations, or processes of different ones of the methods described above may also be executed by programmed computers, processors or other programmable hardware components. Examples may also cover program storage devices, such as digital data storage media, which are machine-, processor- or computer-readable and encode and/or contain machine-executable, processor-executable or computer-executable programs and instructions. Program storage devices may include or be digital storage devices, magnetic storage media such as magnetic disks and magnetic tapes, hard disk drives, or optically readable digital data storage media, for example. Other examples may also include computers, processors, control units, (field) programmable logic arrays ((F)PLAs), (field) programmable gate arrays ((F)PGAs), graphics processor units (GPU), application-specific integrated circuits (ASICs), integrated circuits (ICs) or system-on-a-chip (SoCs) systems programmed to execute the steps of the methods described above.

It is further understood that the disclosure of several steps, processes, operations, or functions disclosed in the description or claims shall not be construed to imply that these operations are necessarily dependent on the order described, unless explicitly stated in the individual case or necessary for technical reasons. Therefore, the previous description does not limit the execution of several steps or functions to a certain order. Furthermore, in further examples, a single step, function, process, or operation may include and/or be broken up into several sub-steps, -functions, -processes or -operations.

If some aspects have been described in relation to a device or system, these aspects should also be understood as a description of the corresponding method. For example, a block, device or functional aspect of the device or system may correspond to a feature, such as a method step, of the corresponding method. Accordingly, aspects described in relation to a method shall also be understood as a description of a corresponding block, a corresponding element, a property or a functional feature of a corresponding device or a corresponding system.

The following claims are hereby incorporated in the detailed description, wherein each claim may stand on its own as a separate example. It should also be noted that although in the claims a dependent claim refers to a particular combination with one or more other claims, other examples may also include a combination of the dependent claim with the subject matter of any other dependent or independent claim. Such combinations are hereby explicitly proposed, unless it is stated in the individual case that a particular combination is not intended. Furthermore, features of a claim should also be included for any other independent claim, even if that claim is not directly defined as dependent on that other independent claim.

**Claims**

1. A method for training at least one machine-learning model for modelling kinetic aspects of a biological organism, the method comprising:

    Training (210) the machine-learning model based on training data, wherein the training data is based on experimental data of a plurality of clones of the biological organism, the training data comprising a subset of training data that is based on experimental data of a single clone,
    wherein a first component of the at least one machine-learning model is trained (211) using the training data, the first component representing a generic kinetic behavior of the biological organism, and
    wherein a second component of the at least one machine-learning model is trained (212) using the subset of the training data, the second component representing a clone-specific kinetic behavior of the biological organism.

2. The method according to claim 1, wherein the training data comprises training input data and training output data, the training input data comprising a representation of an experimental environment of the organism, and the training output data representing kinetic properties observed in response to the respective experimental environment.

3. The method according to claim 2, wherein training the at least one machine-learning model comprises determining a deviation between an output of a function and the training output data, with the function being based on the at least one machine-learning model, a first set of flux modes representing generic functionality of the plurality of clones of the biological organism and a second set of flux modes specific to the single clone of the biological organism.

4. The method according to one of the claims 2 or 3, wherein the representation of the experimental environment corresponds to a compressed representation of the experimental environment having a reduced dimensionality compared to an uncompressed representation of the experimental environment.

5. The method according to one of the claims 1 to 4, wherein the training data is based on experimental data of a plurality of clones of the same cell-line of the biological organism, or wherein the training data is based on experimental data of a plurality of clones of a plurality of different cell-lines of the biological organism.

6. The method according to one of the claims 1 to 5, wherein the training data is based on experimental data from a plurality of different process scales.

7. The method according to one of the claims 1 to 6, wherein the at least one machine-learning model further comprises a third component taking an output of the first and second component at its inputs, the method comprising training the third component of the at least one machine-learning model using the training data, and/or wherein the at least one machine-learning model further comprises a fourth component representing one or more flux modes not represented by the first and/or second component, the method comprising training the fourth machine-learning model using the training data.

8. The method according to one of the claims 1 to 7, wherein the at least one machine-learning model forms a set of machine-learning models, the method comprising training a plurality of sets of machine-learning models, with the plurality of sets of machine-learning models being trained with different seed values, the different seed values affecting at least one of a random initialization of parameters and a dropout of the respective machine-learning models.

9. The method according to one of the claims 1 to 8, wherein the method further comprises adapting (230), using transfer learning, at least the clone-specific second component of the least one machine-learning model based on training data that is based on experimental data of a further single clone.

10. The method according to one of the claims 1 to 9, wherein the method further comprises generating (220) a Digital Twin of the biological organism using the trained at least one machine-learning model.

**11.** The method according to claim 10, further comprising determining (230) a plurality of experiments to be performed using the biological organism, and continuing training (210; 211; 212; 213; 214) of the at least one machine-learning model based on further training data that is based on the plurality of experiments.

**12.** A method for determining at least one target parameter of at least one bioreactor comprising at least one biological organism, the method comprising:
Determining (310) the at least one target parameter using at least one Digital Twin of at least one biological organism that is generated according to claim 10 and at least one corresponding cost function.

**13.** The method according to claim 12, wherein the at least one target parameter is commonly determined for at least two biological organisms using at least two Digital Twins of the at least two biological organisms.

**14.** A method for selecting a clone of a biological organism, the method comprising:

Generating (220) a plurality of Digital Twins of a plurality of clones of the biological organism using the method according to claim 10; and
Selecting (910) the clone by comparing one or more properties of the plurality of Digital Twins.

**15.** A method for controlling a biological manufacturing process involving a biological organism, the method comprising:

Continuously (610) adapting an environment of the biological manufacturing process using a Digital Twin of the biological organism that is generated according to claim 10, with the Digital Twin being supplied with information on the environment of the biological organism using a receding horizon approach; and
Comparing (620) an estimated state of the biological manufacturing process with a defined reference state trajectory of the biological manufacturing process.

**16.** A computer system (20) comprising processing circuitry (24) and storage circuitry (28), the computer system being configured to perform at least one of the method of one of the claims 1 to 11, the method of one of the claims 12 or 13, the method of claim 14and the method of claim 15.

**17.** A computer program having a program code for performing at least one of the method of one of the claims 1 to 11, the method of one of the claims 12 or 13, the method of claim 14and the method of claim 15 when the computer program is executed on a computer, a processor, or a programmable hardware component.

```
┌─────────────────────┐
│  Flux distributions │
│   V₁, V₂, ..., Vₙ   │
│                     │
│         set         │
│      Vᵢ = V₁        │
└─────────────────────┘  ⌐ 110
```

Flux distributions $V_1, V_2, ..., V_n$

set $V_i = V_1$ — 110

Set $rest\_flux = V_i$ — 120

$rest\_flux$ fulfills stop criterion — 130

no → Run decomposition algorithm on $rest\_flux$ to obtain an EFM — 140

→ Include the new EFM into stack of already found EMFs — 150

→ Subtract the new EFM from $rest\_flux$ — 160

yes ↓

$V_i = V_n$ — 170

no → Set $V_i = V_i + 1$ — 180

yes ↓

End Flux decomposition and return stack of found EFMs as the $M$ matrix — 190

Fig. 1

TRAINING AT LEAST ONE MACHINE-LEARNING MODEL

⌐ 210

| TRAINING A FIRST COMPONENT | TRAINING A SECOND COMPONENT |
|---|---|
| ⌐ 211 | ⌐ 212 |

## Fig. 2a

CONTINUE TRAINING

TRAINING AT LEAST ONE MACHINE-LEARNING MODEL

⌐ 210

| TRAINING A FIRST COMPONENT | TRAINING A SECOND COMPONENT | TRAINING A THIRD COMPONENT | TRAINING A FOURTH COMPONENT |
|---|---|---|---|
| ⌐ 211 | ⌐ 212 | ⌐ 213 | ⌐ 214 |

GENERATING A DIGITAL TWIN

⌐ 220

DETERMINING EXPERIMENTS TO BE PERFORMED

⌐ 230

ADAPTING AT LEAST ONE CLONE-SPECIFIC SECOND COMPONENT USING TRANSFER LEARNING

⌐ 240

## Fig. 2b

| MEMORY CIRCUITRY | STORAGE CIRCUITRY |
|---|---|

26

28

PROCESSING CIRCUITRY

24

INTERFACE CIRCUITRY

22

⌐ 20

TRAINING DATA, FLUX MODES

TRAINED ML MODEL OR MODELS, DIGITAL TWIN

## Fig. 2c

GENERATING AT LEAST ONE DIGITAL TWIN

220

DETERMINING EXPERIMENTS TO BE PERFORMED

230

DETERMINING AT LEAST ONE TARGET PARAMETER

310

Fig. 3

GENERATING A DIGITAL TWIN

220

DETERMINING AN ESTIMATED STATE

410

COMPARING THE ESTIMATED STATE
WITH AN OBSERVED STATE

420

Fig. 4

GENERATING A DIGITAL TWIN

220

CONTINUOUSLY ADAPTING AN ENVIRONMENT OF A
BIOLOGICAL MANUFACTURING PROCESS

610

DETERMINING AN ESTIMATED STATE

410

COMPARING AN ESTIMATED STATE OF THE BIOL. MAN.
PROCESS WITH A DEFINED REFERENCE STATE TRAJECTORY

620

Fig. 6

GENERATING DIGITAL TWINS

220

DETERMINING AT LEAST ONE TARGET PARAMETER

310

SELECTING A CLONE

910

Fig. 9

Fig. 5b

Fig. 5a

Fig. 7b

Fig. 7a

State estimation from day 5 - day 12

Model predictive control from day 12 - day 17

Fig. 8a

Fig. 8b

Fig. 10

State estimation for improved predictive quality

Biomass · L-Glutamate · L-Glutamine · L-Lactate · NH4 · Pyruvate · beta-D-Glucose · glycoprotein

■ Measurements with state estimation and control
- - DT Predictions
—— State estimation based DT predictions

Fig. 11

Control applied on a defective batch

**Fig. 12**

■ Measurements with state estimation and control
▲ Measurements without state estimation and control
— Golden batch

Europäisches
Patentamt

European
Patent Office

Office européen
des brevets

# EUROPEAN SEARCH REPORT

**Application Number**

EP 22 18 0917

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/005187 A1 (COSTELLO ZACHARY [US] ET AL) 3 January 2019 (2019-01-03) | 15-17 | INV. C12M1/36 |
| Y | * the whole document * | 15-17 | G06N3/04 |
| A | * paragraphs [0112] – [0119] * <br> * figures 13-15 * <br> * claims 1-29 * | 1-14 | G06N3/08 <br> G05B13/02 <br> G16B5/00 <br> G16B40/00 |
| Y,D | WO 2020/224779 A1 (INSILICO BIOTECHNOLOGY AG [DE]) 12 November 2020 (2020-11-12) | 15 | |
| A | * the whole document * | 1-14,16, 17 | |
| Y | UDUGAMA I. A. ET AL: "Towards Digitalization in Bio-Manufacturing Operations: A Survey on Application of Big Data and Digital Twin Concepts in Denmark", FRONTIERS IN CHEMICAL ENGINEERING, vol. 3, 727152, 16 September 2021 (2021-09-16), pages 1-14, XP093002492, DOI: 10.3389/fceng.2021.727152 | 15-17 | |
| A | * the whole document * <br> * abstract * | 1-14 | TECHNICAL FIELDS SEARCHED (IPC) <br><br> C12M <br> G06N <br> G05B <br> G06F <br> G16B |

-/--

The present search report has been drawn up for all claims

| Place of search | Date of completion of the search | Examiner |
|---|---|---|
| The Hague | 30 November 2022 | van de Kamp, Mart |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

page 1 of 4

Europäisches
Patentamt

European
Patent Office

Office européen
des brevets

# EUROPEAN SEARCH REPORT

Application Number

EP 22 18 0917

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | UDUGAMA I. A. ET AL: "Digital Twin in biomanufacturing: challenges and opportunities towards its implementation", SYSTEMS MICROBIOLOGY AND BIOMANUFACTURING, vol. 1, no. 3, 1 March 2021 (2021-03-01), pages 257-274, XP093002497, ISSN: 2662-7655, DOI: 10.1007/s43393-021-00024-0 Retrieved from the Internet: URL:https://link.springer.com/article/10.1 007/s43393-021-00024-0/fulltext.html> | 15-17 | |
| A | * the whole document * * abstract; figure 1 * * Constitutive elements of a Digital Twin in biomanufacturing; page 263 - page 266 * * Case study: second-generation ethanol fermentation; page 266 - page 269 * | 1-14 | |
| | ----- | | |
| T | HARTMANN F. S. F. ET AL: "Digital models in biotechnology: Towards multi-scale integration and implementation", BIOTECHNOLOGY ADVANCES, vol. 60, 108015, 1 July 2022 (2022-07-01), pages 1-21, XP087185609, ISSN: 0734-9750, DOI: 10.1016/J.BIOTECHADV.2022.108015 [retrieved on 2022-07-01] * the whole document * * 4.1 Digital twins * | 1-17 | TECHNICAL FIELDS SEARCHED (IPC) |
| | ----- | | |
| | -/-- | | |

The present search report has been drawn up for all claims

| Place of search | Date of completion of the search | Examiner |
|---|---|---|
| The Hague | 30 November 2022 | van de Kamp, Mart |

EPO FORM 1503 03.82 (P04C01)

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | MOWBRAY M. ET AL: "Machine learning for biochemical engineering: A review", BIOCHEMICAL ENGINEERING JOURNAL, vol. 172, 108054, 7 May 2021 (2021-05-07), pages 1-22, XP086583248, ISSN: 1369-703X, DOI: 10.1016/J.BEJ.2021.108054 [retrieved on 2021-05-07] | 15-17 | |
| A | * the whole document * <br> * abstract * <br> * 3.1 Bioreactor engineering * <br> * 3.6 Metabolic engineering * <br> * 3.7 Cell culture and protein engineering * <br> * 4.3 Integrating bioprocess knowledge * <br> * 4.4 Novel machine learning algorithms * <br> * page 19, left-hand column, lines 25-29 * | 1-14 | |
| A,D | HEFZI H. ET AL: "A Consensus Genome-scale Reconstruction of Chinese Hamster Ovary Cell Metabolism", CELL SYSTEMS, vol. 3, no. 5, 23 November 2016 (2016-11-23), pages 434-443-e1-e8, XP009193107, ISSN: 2405-4712, DOI: 10.1016/J.CELS.2016.10.020 * the whole document * | 1-17 | TECHNICAL FIELDS SEARCHED (IPC) |

-/--

The present search report has been drawn up for all claims

| Place of search | Date of completion of the search | Examiner |
|---|---|---|
| The Hague | 30 November 2022 | van de Kamp, Mart |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

page 3 of 4

Europäisches
Patentamt

European
Patent Office

Office européen
des brevets

# EUROPEAN SEARCH REPORT

Application Number

**EP 22 18 0917**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WILKENS C. A. ET AL: "Comparative Metabolic Analysis of CHO Cell Clones Obtained through Cell Engineering, for IgG Productivity, Growth and Cell Longevity", PLOS ONE, vol. 10, no. 3, E0119053, 13 March 2015 (2015-03-13), pages 1-15, XP093003736, DOI: 10.1371/journal.pone.0119053 | 15-17 | |
| A | * the whole document * <br> * abstract * <br> ----- | 1-14 | |

TECHNICAL FIELDS
SEARCHED (IPC)

The present search report has been drawn up for all claims

| Place of search | Date of completion of the search | Examiner |
|---|---|---|
| The Hague | 30 November 2022 | van de Kamp, Mart |

EPO FORM 1503 03.82 (P04C01)

page 4 of 4

## ANNEX TO THE EUROPEAN SEARCH REPORT
## ON EUROPEAN PATENT APPLICATION NO.

EP 22 18 0917

This annex lists the patent family members relating to the patent documents cited in the above-mentioned European search report.
The members are as contained in the European Patent Office EDP file on
The European Patent Office is in no way liable for these particulars which are merely given for the purpose of information.

30-11-2022

| Patent document cited in search report | | Publication date | Patent family member(s) | | Publication date |
|---|---|---|---|---|---|
| US 2019005187 | A1 | 03-01-2019 | NONE | | |
| WO 2020224779 | A1 | 12-11-2020 | CN | 114502715 A | 13-05-2022 |
| | | | EP | 3966310 A1 | 16-03-2022 |
| | | | JP | 2022537799 A | 30-08-2022 |
| | | | SG | 11202112113P A | 29-11-2021 |
| | | | US | 2022213429 A1 | 07-07-2022 |
| | | | WO | 2020224779 A1 | 12-11-2020 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2020224779 A1

**Non-patent literature cited in the description**

- **CHAN, S.H.J. ; JI, P.** Decomposing flux distributions into elementary flux modes in genome-scale metabolic networks. *Bioinformatics,* 2011, vol. 27, 2256-2262

- **MORABITO, BRUNO et al.** Multi-mode Model Predictive Control and Estimation for Uncertain Biotechnological Processes. *IFAC-PapersOnLine,* 2019, vol. 52 (1), 709-714
- **HEFZI, HOOMAN et al.** A consensus genome-scale reconstruction of Chinese hamster ovary cell metabolism. *Cell systems,* 2016, vol. 3 (5), 434-443